# EUROPÄISCHE PATENTSCHRIFT

(11) **EP 0 772 610 B1**
(45) Veröffentlichungstag und Bekanntmachung des Hinweises auf die Patenterteilung: **04.10.2000**
(21) Anmeldenummer: 95926953.1
(22) Anmeldetag: 19.07.1995
(51) Int. Cl.: C07D 401/12, C07D 307/94, C07D 307/79, A61K 31/34, A61K 31/44

(54) **DIHYDROBENZOFURANE**
DIHYDROBENZOFURANES
DIHYDROBENZOFURANE

(30) Priorität: 22.07.1994 CH 232394
(43) Veröffentlichungstag der Anmeldung: 14.05.1997
(73) Patentinhaber: Byk Gulden Lomberg Chemische Fabrik GmbH, 78467 Konstanz (DE)
(72) Erfinder: AMSCHLER, Hermann, D-78315 Radolfzell (DE); FLOCKERZI, Dieter, D-78476 Allensbach (DE); GUTTERER, Beate, D-78476 Allensbach (DE); HATZELMANN, Armin, D-78467 Konstanz (DE); SCHUDT, Christian, D-78464 Konstanz (DE); BEUME, Rolf, D-78465 Konstanz (DE); HÄFNER, Dietrich, D-78464 Konstanz (DE); KLEY, Hans-Peter, D-78476 Allensbach (DE); ULRICH, Wolf-Rüdiger, D-78464 Konstanz (DE); THIBAUT, Ulrich, D-78462 Konstanz (DE)
(86) Internationale Anmeldenummer: EP9502841
(87) Internationale Veröffentlichungsnummer: WO9603399

(56) Entgegenhaltungen:
- EP-A- 0 497 564
- WO-A-93/25517

## Beschreibung

### Anwendungsgebiet der Erfindung

Die Erfindung betrifft neue Verbindungen, die in der pharmazeutischen Industrie zur Herstellung von Medikamenten verwendet werden.

### Bekannter technischer Hintergrund

In der internationalen Patentanmeldung W092/12961 werden Benzamide mit PDE-hemmenden Eigenschaften beschrieben. - In der internationalen Patentanmeldung W093/25517 werden trisubstituierte Phenylderivate als selektive PDE-IV-Hemmer offenbart. - In der internationalen Patentanmeldung W094/02465 werden Inhibitoren der c-AMP Phosphodiesterase und des TNF beschrieben.

### Beschreibung der Erfindung

Es wurde nun gefunden, daß sich die nachfolgend näher beschriebenen Benzamide, die sich von den vorveröffentlichten Verbindungen durch eine völlig andersartige Substitution an den Positionen 2 und 3 am Benzamid unterscheiden, überraschende und besonders vorteilhafte Eigenschaften besitzen.

Gegenstand der Erfindung sind somit Verbindungen der Formel I (siehe beigefügtes Formelblatt I), worin
- R1: 1-6C-Alkoxy, 3-7C-Cycloalkoxy, 3-7C-Cycloalkylmethoxy, Benzyloxy oder ganz oder teilweise durch Fluor substituiertes 1-4C-Alkoxy bedeutet,
- R2: 1-4C-Alkyl und
- R3: Wasserstoff oder 1-4C-Alkyl bedeutet,
oder
- R2 und R3: gemeinsam und unter Einschluß der beiden Kohlenstoffatome, an die sie gebunden sind, einen 5-, 6- oder 7-gliedrigen, gewünschtenfalls durch ein Sauerstoffatom unterbrochenen Kohlenwasserstoffring darstellen,
- R4: Phenyl, Pyridyl, durch R41, R42 und R43 substituiertes Phenyl oder durch R44, R45, R46 und R47 substituiertes Pyridyl bedeutet, wobei
R41 Hydroxy, Halogen, Cyano, Carboxyl, Trifluormethyl, 1-4C-Alkyl, 1-4C-Alkoxy, 1-4C-Alkoxycarbonyl, 1-4C-Alkylcarbonyl, 1-4C-Alkylcarbonyloxy, Amino, Mono- oder Di-1-4C-alkylamino oder 1-4C-Alkylcarbonylamino,
R42 Wasserstoff, Hydroxy, Halogen, Amino, Trifluormethyl, 1-4C-Alkyl oder 1-4C-Alkoxy,
R43 Wasserstoff, Halogen, 1-4C-Alkyl oder 1-4C-Alkoxy,
R44 Hydroxy, Halogen, Cyano, Carboxyl, 1-4C-Alkyl, 1-4C-Alkoxy, 1-4C-Alkoxycarbonyl oder Amino,
R45 Wasserstoff- Halogen, Amino oder 1-4C-Alkyl,
R46 Wasserstoff oder Halogen und
R47 Wasserstoff oder Halogen bedeutet,
die Salze dieser Verbindungen sowie die N-Oxide der Pyridine und deren Salze.

1-6C-Alkoxy steht für einen Rest, der neben dem Sauerstoffatom einen geradkettigen oder verzweigten Alkylrest mit 1 bis 6 Kohlenstoffatomen enthält. Als Alkylreste mit 1 bis 6 Kohlenstoffatomen seien hierbei beispielsweise genannt der Hexyl-, Isohexyl- (2-Methylpentyl-), Neohexyl- (2,2-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Cycloalkoxy steht beispielsweise für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder teilweise durch Fluor substituiertes 1-4C-Alkoxy seien beispielsweise der 1,2,2-Trifluorethoxy-, der 2,2,3,3,3-Pentafluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

Als 5-, 6- oder 7-gliedriger, gewünschtenfalls durch ein Sauerstoffatom unterbrochener Kohlenwasserstoffring sei der Cyclopentan-, der Cyclohexan-, der Cycloheptan-, der Tetrahydrofuran- und der Tetrahydropyranring genannt. Wenn R2 und R3 gemeinsam und unter Einschluß der beiden Kohlenstoffatome, an die sie gebunden sind, einen 5-, 6- oder 7-gliedrigen Ring bilden, so liegt eine Spiroverbindung vor.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

Halogen im Sinne der vorliegenden Erfindung ist Brom, Chlor und Fluor.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

1-4C-Alkoxycarbonyl steht für eine Carbonylgruppe, an die einer der vorstehend genannten 1-4C-Alkoxyreste gebunden ist. Beispielsweise seien der Methoxycarbonyl- (CH₃O-CO-) und der Ethoxycarbonylrest (CH₃CH₂O-CO-) genannt.

1-4C-Alkylcarbonyl steht für eine Carbonylgruppe, an die einer der vorstehend genannten 1-4C-Alkylreste gebunden ist. Beispielsweise sei der Acetylrest (CH₃CO-) genannt.

1-4C-Alkylcarbonyloxyreste enthalten neben dem Sauerstoffatom einen der vorstehend genannten 1-4C-Alkylcarbonylreste. Beispielsweise sei der Acetoxyrest (CH₃CO-O-) genannt.

Als Mono- oder Di-1-4C-alkylaminoreste seien beispielsweise der Methylamino-, der Dimethylamino- und der Diethylaminorest genannt.

Als 1-4C-Alkylcarbonylaminorest sei beispielsweise der Acetylamidorest (-NH-CO-CH₃) genannt.

Als beispielhafte, durch R41, R42 und R43 substituierte Phenylreste seien die Reste 2-Acetylphenyl, 2-Aminophenyl, 2-Bromphenyl, 2-Chlorphenyl, 2,3-Dichlorphenyl, 2,4-Dichlorphenyl, 4-Diethylamino-2-methylphenyl, 4-Brom-2-trifluormethylphenyl, 2-Carboxy-5-chlorphenyl, 3,5-Dichlor-2-hydroxyphenyl, 2-Brom-4-carboxy-5-hydroxyphenyl, 2,6-Dichlorphenyl, 2,5-Dichlorphenyl, 2,4,6-Trichlorphenyl, 2,4,6-Trifluorphenyl, 2,6-Dibromphenyl, 2-Cyanphenyl, 4-Cyan-2-fluorphenyl, 2-Fluorphenyl, 2,4-Difluorphenyl, 2,6-Difluorphenyl, 2-Chlor-6-fluorphenyl, 2-Hydroxyphenyl, 2-Hydroxy-4-methoxyphenyl, 2,4-Dihydroxyphenyl, 2-Methoxyphenyl, 2,3-Dimethoxyphenyl, 2,4-Dimethoxyphenyl, 2,6-Dimethoxyphenyl, 2-Dimethylaminophenyl, 2-Methylphenyl, 2-Chlor-6-methylphenyl, 2,4-Dimethylphenyl, 2,6-Dimethylphenyl, 2,3-Dimethylphenyl, 2-Methoxycarbonylphenyl, 2-Trifluormethylphenyl, 2,6-Dichlor-4-methoxyphenyl, 2,6-Dichlor-4-cyanphenyl, 2,6-Dichlor-4-aminophenyl, 2,6-Dichlor-4-methoxycarbonylphenyl, 4-Acetylamino-2,6-dichlorphenyl und 2,6-Dichlor-4-ethoxycarbonylphenyl genannt.

Als beispielhafte, durch R44, R45, R46 und R47 substituierte Pyridylreste seien die Reste 3,5-Dichlorpyrid-4-yl, 2,6-Diaminopyrid-3-yl, 4-Aminopyrid-3-yl, 3-Methylpyrid-2-yl, 4-Methylpyrid-2-yl, 5-Hydroxypyrid-2-yl, 4-Chlorpyrid-3-yl, 3-Chlorpyrid-2-yl, 3-Chlorpyrid-4-yl, 2-Chlorpyrid-3-yl, 2,3,5,6-Tetrafluorpyrid-4-yl, 3,5-Dichlor-2,6-difluorpyrid-4-yl, 3,5-Dibrompyrid-2-yl, 3,5-Dibrompyrid-4-yl, 3,5-Dichlorpyrid-4-yl, 2,6-Dichlorpyrid-3-yl, 3,5-Dimethylpyrid-4-yl, 3-Chlor-2,5,6-trifluorpyrid-4-yl und 2,3,5-Trifluorpyrid-4-yl genannt.

Als Salze kommen für Verbindungen der Formel I - je nach Substitution - alle Säureadditionssalze aber insbesondere alle Salze mit Basen in Betracht. Besonders erwähnt seien die pharmakologisch verträglichen Salze der in der Galenik üblicherweise verwendeten anorganischen und organischen Säuren und Basen. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphtoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen vor allem auch Salze mit Basen in Betracht. Als Beispiele für basische Salze seien Lithium-, Natrium-, Kalium-, Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Hervorzuhebende Verbindungen der Formel I sind solche, in denen
- R1: 1-4C-Alkoxy, 3-5C-Cycloalkoxy, 3-5C-Cycloalkylmethoxy oder ganz oder teilweise durch Fluor substituiertes 1-4C-Alkoxy bedeutet,
- R2: 1-4C-Alkyl und
- R3: Wasserstoff oder 1-4C-Alkyl bedeutet,
oder
- R2 und R3: gemeinsam und unter Einschluß der beiden Kohlenstoffatome, an die sie gebunden sind, einen Cyclopentan-, Cyclohexan-, Tetrahydrofuran-oder Tetrahydropyranring darstellen,
- R4: Phenyl, Pyridyl, durch R41, R42 und R43 substituiertes Phenyl oder durch R44, R45, R46 und R47 substituiertes Pyridyl bedeutet, wobei
R41 Halogen, Cyano, Carboxyl, 1-4C-Alkyl, 1-4C-Alkoxy oder 1-4C-Alkoxycarbonyl,
R42 Wasserstoff, Halogen, 1-4C-Alkyl oder 1-4C-Alkoxy,
R43 Wasserstoff, Halogen, 1-4C-Alkyl oder 1-4C-Alkoxy,
R44 Halogen oder 1-4C-Alkyl,
R45 Wasserstoff oder Halogen,
R46 Wasserstoff oder Halogen und
R47 Wasserstoff oder Halogen bedeutet,
die Salze dieser Verbindungen sowie die N-Oxide der Pyridine und deren Salze.

Besonderes hervorzuhebende Verbindungen der Formel I sind solche, in denen
- R1: 1-4C-Alkoxy, 3-5C-Cycloalkoxy, 3-5C-Cycloalkylmethoxy oder ganz oder teilweise durch Fluor substituiertes 1-4C-Alkoxy bedeutet,
- R2: 1-4C-Alkyl und
- R3: Wasserstoff oder 1-4C-Alkyl bedeutet,
oder
- R2 und R3: gemeinsam und unter Einschluß der beiden Kohlenstoffatome, an die sie gebunden sind, einen Cyclopentan-, Cyclohexan-, Tetrahydrofuran-oder Tetrahydropyranring darstellen,
- R4: 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2-Chlor-6-fluorphenyl, 2,6-Difluorphenyl, 2,6-Dichlorphenyl, 3,5-Dichlorpyrid-4-yl, 3-Methylpyrid-2-yl, 2-Chlorpyrid-3-yl, 3,5-Dibrompyrid-2-yl, 3,5-Difluorpyrid-4-yl, 2-Chlorphenyl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

Bevorzugte Verbindungen der Formel I sind solche, in denen
- R1: Methoxy, Ethoxy, Cyclopropylmethoxy, Difluormethoxy, Trifluormethoxy oder 2,2,2-Trifluorethoxy bedeutet,
- R2: Methyl oder Ethyl und
- R3: Wasserstoff oder Methyl bedeutet,
oder
- R2 und R3: gemeinsam und unter Einschluß der beiden Kohlenstoffatome, an die sie gebunden sind, einen Cyclopentan-, Cyclohexan-, Tetrahydrofuranoder Tetrahydropyranring darstellen,
- R4: 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2-Chlor-6-fluorphenyl, 2,6-Difluorphenyl, 2,6-Dichlorphenyl, 3,5-Dichlorpyrid-4-yl, 3-Methylpyrid-2-yl, 2-Chlorpyrid-3-yl, 3,5-Dibrompyrid-2-yl, 3,5-Difluorpyrid-4-yl, 2-Chlorphenyl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

Besonders bevorzugte Verbindungen der Formel I sind solche, in denen
- R1: Methoxy, Ethoxy, Cyclopropylmethoxy, Difluormethoxy, Trifluormethoxy oder 2,2,2-Trifluorethoxy bedeutet,
- R2: Methyl oder Ethyl und
- R3: Wasserstoff oder Methyl bedeutet,
oder
- R2 und R3: gemeinsam und unter Einschluß der beiden Kohlenstoffatome, an die sie gebunden sind, einen Cyclopentan-, Cyclohexan-, Tetrahydrofuran-oder Tetrahydropyranring darstellen,
- R4: 3,5-Dichlorpyrid-4-yl, 2,6-Dichlorphenyl oder 2,6-Difluorphenyl bedeutet,
die Salze dieser Verbindungen sowie die N-Oxide der Pyridine und deren Salze.

Beispielhafte erfindungsgemäße Verbindungen sind in den folgenden Tabellen aufgeführt:

**Tabelle 1**

| Verbindungen der Formel I (siehe beigefügtes Formelblatt I) mit R4=3,5-Dichlorpyrid-4-yl und den folgenden weiteren Substituentenbedeutungen: | | |
|---|---|---|
| R1 | R2 | R3 |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C₃H₅ | CH₂CH₂-O | |
| OCF₂H | CH₂CH₂-O | |
| OCF₃ | CH₂CH₂-O | |
| OCH₂CF₃ | CH₂CH₂-O | |
| OCH₃ | CH₂CH₂-O-CH₂ | |
| OC₂H₅ | CH₂CH₂-O-CH₂ | |
| OCH₂C₃H₅ | CH₂CH₂-O-CH₂ | |
| OCF₂H | CH₂CH₂-O-CH₂ | |
| OCF₃ | CH₂CH₂-O-CH₂ | |
| OCH₂CF₃ | CH₂CH₂-O-CH₂ | |

**Tabelle 2**

| Verbindungen der Formel I (siehe beigefügtes Formelblatt I) mit R4=2,6-Dichlorphenyl und den folgenden weiteren Substituentenbedeutungen: | | |
|---|---|---|
| R1 | R2 | R3 |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| 0CH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | | CH₂CH₂CH₂CH₂ |
| OCF₃ | | CH₂CH₂CH₂CH₂ |
| OCH₂CF₃ | | CH₂CH₂CH₂CH₂ |
| OCH₃ | | CH₂-O-CH₂ |
| OC₂H₅ | | CH₂-O-CH₂ |
| OCH₂C₃H₅ | | CH₂-O-CH₂ |
| OCF₂H | | CH₂-O-CH₂ |
| OCF₃ | | CH₂-O-CH₂ |
| OCH₂CF₃ | | CH₂-O-CH₂ |
| OCH₃ | | CH₂CH₂-O |
| OC₂H₅ | | CH₂CH₂-0 |
| OCH₂C₃H₅ | | CH₂CH₂-O |
| OCF₂H | | CH₂CH₂-O |
| OCF₃ | | CH₂CH₂-O |
| OCH₂CF₃ | | CH₂CH₂-O |
| OCH₃ | | CH₂CH₂-O-CH₂ |
| OC₂H₅ | | CH₂CH₂-O-CH₂ |
| OCH₂C₃H₅ | | CH₂CH₂-O-CH₂ |
| OCF₂H | | CH₂CH₂-O-CH₂ |
| OCF₃ | | CH₂CH₂-O-CH₂ |
| OCH₂CF₃ | | CH₂CH₂-O-CH₂ |

**Tabelle 3**

| Verbindungen der Formel I (siehe beigefügtes Formelblatt I) mit R4=2,6-Difluorphenyl und den folgenden weiteren Substituentenbedeutungen: | | |
|---|---|---|
| R1 | R2 | R3 |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | CH₂CH₂CH₂ | |
| OC₂H₅ | CH₂CH₂CH₂ | |
| OCH₂C₃H₅ | CH₂CH₂CH₂ | |
| OCF₂H | CH₂CH₂CH₂ | |
| OCF₃ | CH₂CH₂CH₂ | |
| OCH₂CF₃ | CH₂CH₂CH₂ | |
| OCH₃ | CH₂CH₂CH₂CH₂ | |
| OC₂H₅ | CH₂CH₂CH₂CH₂ | |
| OCH₂C₃H₅ | CH₂CH₂CH₂CH₂ | |
| OCF₂H | CH₂CH₂CH₂CH₂ | |
| OCF₃ | CH₂CH₂CH₂CH₂ | |
| OCH₂CF₃ | CH₂CH₂CH₂CH₂ | |
| OCH₃ | CH₂-O-CH₂ | |
| OC₂H₅ | CH₂-O-CH₂ | |
| OCH₂C₃H₅ | CH₂-O-CH₂ | |
| OCF₂H | CH₂-O-CH₂ | |
| OCF₃ | CH₂-O-CH₂ | |
| OCH₂CF₃ | CH₂-O-CH₂ | |
| OCH₃ | CH₂CH₂-O | |
| OC₂H₅ | CH₂CH₂-O | |
| OCH₂C₃H₅ | CH₂CH₂-O | |
| OCF₂H | CH₂CH₂-O | |
| OCF₃ | CH₂CH₂-O | |
| OCH₂CF₃ | CH₂CH₂-O | |
| OCH₃ | | CH₂CH₂-O-CH₂ |
| OC₂H₅ | | CH₂CH₂-O-CH₂ |
| OCH₂C₃H₅ | | CH₂CH₂-O-CH₂ |
| OCF₂H | | CH₂CH₂-O-CH₂ |
| OCF₃ | | CH₂CH₂-O-CH₂ |
| OCH₂CF₃ | | CH₂CH₂-O-CH₂ |

**Tabelle 4**

| Verbindungen der Formel I (siehe beigefügtes Formelblatt I) mit R4=2-Chlorpyrid-3-yl und den folgenden weiteren Substituentenbedeutungen: | | |
|---|---|---|
| R1 | R2 | R3 |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | | CH₂CH₂CH₂ |
| OC₂H₅ | | CH₂CH₂CH₂ |
| OCH₂C₃H₅ | | CH₂CH₂CH₂ |
| OCF₂H | | CH₂CH₂CH₂ |
| OCF₃ | | CH₂CH₂CH₂ |
| OCH₂CF₃ | | CH₂CH₂CH₂ |
| OCH₃ | | CH₂CH₂CH₂CH₂ |
| OC₂H₅ | | CH₂CH₂CH₂CH₂ |
| OCH₂C₃H₅ | | CH₂CH₂CH₂CH₂ |
| OCF₂H | | CH₂CH₂CH₂CH₂ |
| OCF₃ | | CH₂CH₂CH₂CH₂ |
| OCH₂CF₃ | | CH₂CH₂CH₂CH₂ |
| OCH₃ | | CH₂-O-CH₂ |
| OC₂H₅ | | CH₂-O-CH₂ |
| OCH₂C₃H₅ | | CH₂-O-CH₂ |
| OCF₂H | | CH₂-O-CH₂ |
| OCF₃ | | CH₂-O-CH₂ |
| OCH₂CF₃ | | CH₂-O-CH₂ |
| OCH₃ | | CH₂CH₂-O |
| OC₂H₅ | | CH₂CH₂-O |
| OCH₂C₃H₅ | | CH₂CH₂-O |
| OCF₂H | | CH₂CH₂-O |
| OCF₃ | | CH₂CH₂-O |
| OCH₂CF₃ | | CH₂CH₂-O |
| OCH₃ | | CH₂CH₂-O-CH₂ |
| OC₂H₅ | | CH₂CH₂-O-CH₂ |
| OCH₂C₃H₅ | | CH₂CH₂-O-CH₂ |
| OCF₂H | | CH₂CH₂-O-CH₂ |
| OCF₃ | | CH₂CH₂-O-CH₂ |
| OCH₂CF₃ | | CH₂CH₂-O-CH₂ |

**Tabelle 5**

| Verbindungen der Formel I (siehe beigefügtes Formelblatt I) mit R4=2-Chlor-6-methylphenyl und den folgenden weiteren Substituentenbedeutungen: | | |
|---|---|---|
| R1 | R2 | R3 |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | | CH₂CH₂CH₂ |
| OC₂H₅ | | CH₂CH₂CH₂ |
| OCH₂C₃H₅ | | CH₂CH₂CH₂ |
| OCF₂H | | CH₂CH₂CH₂ |
| OCF₃ | | CH₂CH₂CH₂ |
| OCH₂CF₃ | | CH₂CH₂CH₂ |
| OCH₃ | | CH₂CH₂CH₂CH₂ |
| OC₂H₅ | | CH₂CH₂CH₂CH₂ |
| OCH₂C₃H₅ | | CH₂CH₂CH₂CH₂ |
| OCF₂H | | CH₂CH₂CH₂CH₂ |
| OCF₃ | | CH₂CH₂CH₂CH₂ |
| OCH₂CF₃ | | CH₂CH₂CH₂CH₂ |
| OCH₃ | | CH₂-O-CH₂ |
| OC₂H₅ | | CH₂-O-CH₂ |
| OCH₂C₃H₅ | | CH₂-O-CH₂ |
| OCF₂H | | CH₂-O-CH₂ |
| OCF₃ | | CH₂-O-CH₂ |
| OCH₂CF₃ | | CH₂-O-CH₂ |
| OCH₃ | | CH₂CH₂-O |
| OC₂H₅ | | CH₂CH₂-O |
| OCH₂C₃H₅ | | CH₂CH₂-O |
| OCF₂H | | CH₂CH₂-O |
| OCF₃ | | CH₂CH₂-O |
| OCH₂CF₃ | | CH₂CH₂-O |
| OCH₃ | | CH₂CH₂-O-CH₂ |
| OC₂H₅ | | CH₂CH₂-O-CH₂ |
| OCH₂C₃H₅ | | CH₂CH₂-O-CH₂ |
| OCF₂H | | CH₂CH₂-O-CH₂ |
| OCF₃ | | CH₂CH₂-O-CH₂ |
| OCH₂CF₃ | | CH₂CH₂-O-CH₂ |

**Tabelle 6**

| Verbindungen der Formel I (siehe beigefügtes Formelblatt I) mit R4=2-Chlor-6-fluorphenyl und den folgenden weiteren Substituentenbedeutungen: | | |
|---|---|---|
| R1 | R2 | R3 |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | | CH₂CH₂CH₂ |
| OC₂H₅ | | CH₂CH₂CH₂ |
| OCH₂C₃H₅ | | CH₂CH₂CH₂ |
| OCF₂H | | CH₂CH₂CH₂ |
| OCF₃ | | CH₂CH₂CH₂ |
| OCH₂CF₃ | | CH₂CH₂CH₂ |
| OCH₃ | | CH₂CH₂CH₂CH₂ |
| OC₂H₅ | | CH₂CH₂CH₂CH₂ |
| OCH₂C₃H₅ | | CH₂CH₂CH₂CH₂ |
| OCF₂H | | CH₂CH₂CH₂CH₂ |
| OCF₃ | | CH₂CH₂CH₂CH₂ |
| OCH₂CF₃ | | CH₂CH₂CH₂CH₂ |
| OCH₃ | | CH₂-O-CH₂ |
| OC₂H₅ | | CH₂-O-CH₂ |
| OCH₂C₃H₅ | | CH₂-O-CH₂ |
| OCF₂H | | CH₂-O-CH₂ |
| OCF₃ | | CH₂-O-CH₂ |
| OCH₂CF₃ | | CH₂-O-CH₂ |
| OCH₃ | | CH₂CH₂-O |
| OC₂H₅ | | CH₂CH₂-O |
| OCH₂C₃H₅ | | CH₂CH₂-O |
| OCF₂H | | CH₂CH₂-O |
| OCF₃ | | CH₂CH₂-O |
| OCH₂CF₃ | | CH₂CH₂-O |
| OCH₃ | | CH₂CH₂-O-CH₂ |
| OC₂H₅ | | CH₂CH₂-O-CH₂ |
| OCH₂C₃H₅ | | CH₂CH₂-O-CH₂ |
| OCF₂H | | CH₂CH₂-O-CH₂ |
| OCF₃ | | CH₂CH₂-O-CH₂ |
| OCH₂CF₃ | | CH₂CH₂-O-CH₂ |

**Tabelle 7**

| Verbindungen der Formel I (siehe beigefügtes Formelblatt I) mit R4=3,5-Di-fluoropyrid-4-yl und den folgenden weiteren Substituentenbedeutungen: | | |
|---|---|---|
| R1 | R2 | R3 |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | | CH₂CH₂CH₂ |
| OC₂H₅ | | CH₂CH₂CH₂ |
| OCH₂C₃H₅ | | CH₂CH₂CH₂ |
| OCF₂H | | CH₂CH₂CH₂ |
| OCF₃ | | CH₂CH₂CH₂ |
| OCH₂CF₃ | | CH₂CH₂CH₂ |
| OCH₃ | | CH₂CH₂CH₂CH₂ |
| OC₂H₅ | | CH₂CH₂CH₂CH₂ |
| OCH₂C₃H₅ | | CH₂CH₂CH₂CH₂ |
| OCF₂H | | CH₂CH₂CH₂CH₂ |
| OCF₃ | | CH₂CH₂CH₂CH₂ |
| OCH₂CF₃ | | CH₂CH₂CH₂CH₂ |
| OCH₃ | | CH₂-O-CH₂ |
| OC₂H₅ | | CH₂-O-CH₂ |
| OCH₂C₃H₅ | | CH₂-O-CH₂ |
| OCF₂H | | CH₂-O-CH₂ |
| OCF₃ | | CH₂-O-CH₂ |
| OCH₂CF₃ | | CH₂-O-CH₂ |
| OCH₃ | | CH₂CH₂-O |
| OC₂H₅ | | CH₂CH₂-O |
| OCH₂C₃H₅ | | CH₂CH₂-O |
| OCF₂H | | CH₂CH₂-O |
| OCF₃ | | CH₂CH₂-O |
| OCH₂CF₃ | | CH₂CH₂-O |
| OCH₃ | | CH₂CH₂-O-CH₂ |
| OC₂H₅ | | CH₂CH₂-O-CH₂ |
| OCH₂C₃H₅ | | CH₂CH₂-O-CH₂ |
| OCF₂H | | CH₂CH₂-O-CH₂ |
| OCF₃ | | CH₂CH₂-O-CH₂ |
| OCH₂CF₃ | | CH₂CH₂-O-CH₂ |

**Tabelle 8**

| Verbindungen der Formel I (siehe beigefügtes Formelblatt I) mit R4=2-Chlorphenyl und den folgenden weiteren Substituentenbedeutungen: | | |
|---|---|---|
| R1 | R2 | R3 |
| OCH₃ | CH₃ | H |
| OC₂H₅ | CH₃ | H |
| OCH₂C₃H₅ | CH₃ | H |
| OCF₂H | CH₃ | H |
| OCF₃ | CH₃ | H |
| OCH₂CF₃ | CH₃ | H |
| OCH₃ | C₂H₅ | CH₃ |
| OC₂H₅ | C₂H₅ | CH₃ |
| OCH₂C₃H₅ | C₂H₅ | CH₃ |
| OCF₂H | C₂H₅ | CH₃ |
| OCF₃ | C₂H₅ | CH₃ |
| OCH₂CF₃ | C₂H₅ | CH₃ |
| OCH₃ | | CH₂CH₂CH₂ |
| OCF₃ | | CH₂CH₂CH₂ |
| OCH₂CF₃ | | CH₂CH₂CH₂ |
| OCH₃ | | CH₂CH₂CH₂CH₂ |
| OC₂H₅ | | CH₂CH₂CH₂CH₂ |
| OCH₂C₃H₅ | | CH₂CH₂CH₂CH₂ |
| OCF₂H | | CH₂CH₂CH₂CH₂ |
| OC₂H₅ | | CH₂CH₂CH₂ |
| OCH₂C₃H₅ | | CH₂CH₂CH₂ |
| OCF₂H | | CH₂CH₂CH₂ |
| OCF₃ | | CH₂CH₂CH₂CH₂ |
| OCH₂CF₃ | | CH₂CH₂CH₂CH₂ |
| OCH₃ | | CH₂-O-CH₂ |
| OC₂H₅ | | CH₂-O-CH₂ |
| OCH₂C₃H₅ | | CH₂-O-CH₂ |
| OCF₂H | | CH₂-O-CH₂ |
| OCF₃ | | CH₂-O-CH₂ |
| OCH₂CF₃ | | CH₂-O-CH₂ |
| OCH₃ | | CH₂CH₂-O |
| OC₂H₅ | | CH₂CH₂-O |
| OCH₂C₃H₅ | | CH₂CH₂-O |
| OCF₂H | | CH₂CH₂-O |
| OCF₃ | | CH₂CH₂-O |
| OCH₂CF₃ | | CH₂CH₂-O |
| OCH₃ | | CH₂CH₂-O-CH₂ |
| OC₂H₅ | | CH₂CH₂-O-CH₂ |
| OCH₂C₃H₅ | | CH₂CH₂-O-CH₂ |
| OCF₂H | | CH₂CH₂-O-CH₂ |
| OCF₃ | | CH₂CH₂-O-CH₂ |
| OCH₂CF₃ | | CH₂CH₂-O-CH₂ |

sowie die Salze der in den Tabellen genannten Verbindungen.

Bei den Verbindungen der Formel I handelt es sich - sofern die Substitutionen -R2 und -CH₂R3 nicht identisch sind - um chirale Verbindungen. Die Erfindung umfaßt daher sowohl die reinen Enantiomeren als auch deren Gemische in jedem Mischungsverhältnis, einschließlich der Racemate.

Weiterer Gegenstand der Erfindung ist ein Verfahren zur Herstellung der Verbindungen der Formel I und ihrer Salze, sowie der N-Oxide der Pyridine und deren Salze. Das Verfahren ist dadurch gekennzeichnet, daß man Verbindungen der Formel II (siehe beigefügtes Formelblatt I), in denen R1, R2 und R3 die oben angegebenen Bedeutungen haben und X eine geeignete Abgangsgruppe darstellt, mit Aminen R4-NH₂ umsetzt, und daß man gewünschtenfalls anschließend erhaltene Verbindungen der Formel I in ihre Salze und/oder erhaltene Pyridine in die N-Oxide und gewünschtenfalls anschließend in die Salze überführt, oder daß man gewünschtenfalls anschließend erhaltene Salze der Verbindungen der Formel I in die freien Verbindungen überführt.

Welche Abgangsgruppen X geeignet sind, ist dem Fachmann aufgrund seines Fachwissens geläufig. Beispielsweise wird von geeigneten Säurehalogeniden der Formel II (X=Cl oder Br) ausgegangen. Im übrigen erfolgt die Umsetzung beispielsweise so wie in den nachfolgenden Beispielen beschrieben, oder auf eine dem Fachmann an sich vertraute Weise (z.B. so, wie in der internationalen Patentanmeldung WO 92/12961 beschrieben).

Die N-Oxidation erfolgt auf eine dem Fachmann ebenfalls vertraute Weise, z.B. mit Hilfe von m-Chlorperoxibenzoesäure in Dichlormethan bei Raumtemperatur. Welche Reaktionsbedingungen für die Durchführung des Verfahrens im einzelnen erforderlich sind, ist dem Fachmann aufgrund seines Fachwissens geläufig.

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die Verbindungen der Formel II können gemäß dem allgemeinen Reaktionsschema auf dem beigefügten Formelblatt II hergestellt werden. Beispielhaft ist die Herstellung von Verbindungen der Formel II in den nachfolgenden Beispielen unter "Ausgangsverbindungen" beschrieben. Die Herstellung weiterer Verbindungen der Formel II kann auf analoge Weise erfolgen.

Die Amine R4-NH₂ sind bekannt, oder sie können auf bekannte Weise hergestellt werden.

Die folgenden Beispiele erläutern die Erfindung näher, ohne sie einzuschränken.

Die Abkürzung RT steht für Raumtemperatur, h steht für Stunde(n), min für Minute(n), Fp. für Schmelzpunkt.

### Beispiele

### Endprodukte

### 1. 2,3-Dihydro-2,2-dimethyl-7-methoxybenzofuran-4-carbonsäure-N-3,5-dichlor-4-pyridyl-amid

0,22 g Natriumhydrid (80 % in Paraffin) werden in 20 ml wasserfreiem THF suspendiert und dann unter Rühren eine Lösung von 0,5 g 4-Amino-3,5-dichlorpyridin in 5 ml abs. THF zugetropft. Es wird 30 min gerührt und dann eine Lösung von 2,3-Dihydro-2,2-dimethyl-7-methoxy-benzofuran-4-carbonsäurechlorid (hergestellt aus 0,8 g 2,3-Dihydro-2,2-dimethyl-7-methoxy-benzofuran-4-carbonsäure, siehe Beispiel A1) in 10 ml abs. THF zugetropft. Nach 10 min wird auf Wasser gegossen, mit 2N HCl pH 4 eingestellt, 3 x mit Ethylacetat extrahiert, die vereinigten Extrakte über Natriumsulfat getrocknet und filtriert. Der nach Einengen am Rotationsverdampfer verbleibende ölige Rückstand wird über eine Kieselgelsäule mit Dichlormethan/Methanol (98:2) chromatographiert. Die chromatographisch reinen Fraktionen werden vereinigt, eingeengt und mit Diethylether kristallisiert. Man erhält 0,7 g der Titelverbindung mit Fp. 140-142°C.

### 2. 2,3-Dihydro-7-methoxybenzofuran-2-spiro-1'-cyclopentan-4-carbonsäure-N-3,5-dichlor-4-pyridyl-amid

Analog Beispiel 1 erhält man aus 0,55 g Natriumhydrid (80 % in Paraffin) in 50 ml abs. THF, 1,5 g 4-Amino-3,5-dichlorpyridin in 20 ml abs. THF und 2,5 g 2,3-Dihydro-7-methoxybenzofuran-2-spiro-1'-cyclopentan-4-carbonsäurechlorid 1,4 g der Titelverbindung mit Fp. 168-170°C (aus Diethylether).

### 3. 7-Difluormethoxy-2,3-dihydrobenzofuran-2-spiro-1'-cyclopentan-4-carbonsäure-N-3,5-dichlor-4-pyridyl-amid

Analog Beispiel 1 erhält man aus 0,15 g Natriumhydrid (80 % in Paraffin) in 20 ml abs. THF, 0,41 g 4-Amino-3,5-dichlorpyridin in 10 ml abs. THF und einer Lösung von 7-Difluormethoxy-2,3-dihydrobenzofuran-2-spiro-1-cyclopentan-4-carbonsäure-chlorid (hergestellt aus 0,7 g 7-Difluormethoxy-2,3-dihydrobenzofuran-2-spiro-1'-cyclopentan-4-carbonsäure, siehe Beispiel C1) in 10 ml abs. THF nach Chromatografie (Kieselgel, Laufmittel: Ethylacetat/Petrolether 4:6) 0,15 g der Titelverbindung mit Fp:. 152-153°C (aus Diisopropylether).

### 4. 2,3-Dihydro-7-methoxybenzofuran-2-spiro-1'-cyclohexan-4-carbonsäure-N-3,5-dichlor-4-pyridylamid

Analog Beispiel 1 erhält man aus 0,46 g Natriumhydrid (80 % in Paraffin) in 20 ml abs. THF, 1,24 g 4-Amino-3,5-dichlorpyridin in 20 ml abs. THF und einer Lösung von 2,3-Dihydro-7-methoxy-benzofuran-2-spiro-1'-cyclohexan-4-carbonsäurechlorid (hergestellt aus 2 g 2,3-Dihydro-7-methoxybenzofuran-2-spiro-1'-cyclohexan-4-carbonsäure, siehe Beispiel D1) in 20 ml abs. THF 2,9 g der Titelverbindung mit Fp.: 169-170°C.

### 5. 2,3-Dihydro-7-methoxybenzofuran-2-spiro-1'-(4'-oxacyclohexan)-4-carbonsäure-N-3,5-dichlor-4-pyridylamid

Analog Beispiel 1 erhält man aus 0,22 g Natriumhydrid in 40 ml abs. THF, 0,62 g 4-Amino-3,5-dichlorpyridin in 20 ml abs. THF und einer Lösung von 2,3-Dihydro-7-methoxybenzofuran-2-spiro-1'-(4'-oxacyclohexan)-4-carbonsäurechlorid (hergestellt aus 1 g 2,3-Dihydro-7-methoxybenzofuran-2-spiro-1'-(4'-oxacyclohexan)-4-carbonsäure, siehe Beispiel E1) in 10 ml abs. THF 0,3 g der Titelverbindung mit Fp.: 208-210°C.

### 6. 2,2-Diethyl-2,3-dihydro-7-methoxybenzofuran-4-carbonsäure-N-3,5-dichlor-4-pyridyl-amid

Analog Beispiel 1 erhält man aus 0,3 g Natriumhydrid (80 % in Paraffin) in 20 ml abs. THF, 0,8 g 4-Amino-3,5-dichlorpyridin in 10 ml abs. THF und einer Lösung von 2,2-Diethyl-2,3-dihydro-7-methoxybenzofurancarbonsäurechlorid (hergestellt aus 1,2 g 2,2-Diethyl-2,3-dihydro-7-methoxybenzofuran-4-carbonsäure, siehe Beispiel F1) in 20 ml THF nach Chromatografie (Kieselgel, Dichlormethan, Methanol 98:2) 0,9 g der Titelverbindung mit Fp.: 171-172°C.

### 7. 2,3-Dihydro-7-methoxybenzofuran-2-spiro-1'-cyclopentan-4-carbonsäure-2,6-dichloranilid

Eine Lösung von 0,65 g 2,6-Dichloranilin und 0,7 ml Triethylamin in 20 ml Dioxan wird auf 40-50°C erwärmt und dann eine Lösung von 2,3-Dihydro-7-methoxybenzofuran-2-spiro-1'-cyclopentan-4-carbonsäurechlorid (hergestellt aus 1 g 2,3-Dihydro-7-methoxybenzofuran-2-spiro-1'-cyclopentan-4-carbonsäure, siehe Beispiel A1) in 10 ml Dioxan zugetropft. Es wird 1 h bei 50°C gerührt, dann wird auf Wasser gegossen und mit Ethylacetat extrahiert. Der organische Extrakt wird über Natriumsulfat getrocknet, eingeengt und der Rückstand über eine Kieselgelsäule mit Ethylacetat/Petrolether 4:6 chromatografiert. Die chromatografisch reinen Fraktionen werden vereinigt, eingeengt und mit Diisopropylether kristallisiert. Man erhält 0,2 g der Titelverbindung mit Fp.: 172-174°C.

### 8. 2,3-Dihydro-7-methoxybenzofuran-2-spiro-1'-cyclopentan-4-carbonsäure-2,6-difluoranilid

Analog Beispiel 7 erhält man aus 0,65 ml 2,6-Difluoranilin, 0,9 ml Triethylamin und 2,3-Dihydro-7-methoxybenzofuran-2-spiro-1'-cyclopentan-4-carbonsäurechlorid (hergestellt aus 1,5 g Carbonsäure siehe Beispiel A1) 1,2 g der Titelverbindung mit Fp.: 142-145°C (aus Diisopropylether).

### Ausgangsverbindungen

### A1: 2,3-Dihydro-2,2-dimethyl-7-methoxybenzofuran-4-carbonsäurechlorid

0,8 g 2,3-Dihydro-2,2-dimethyl-7-methoxybenzofuran-4-carbonsäure werden in einem Gemisch aus 50 ml abs. Toluol und 3 ml Thionylchlorid 1 h lang unter Rückfluß zum Sieden erhitzt. Das Lösungsmittel wird am Rotationsverdampfer abdestilliert und dann noch 2 x Toluol (ca. 30 ml) zugegeben und wieder eingeengt. Der Rückstand wird im Hochvakuum getrocknet und ohne weitere Reinigung bei Beispiel 1 eingesetzt.

### A2: 2,3-Dihydro-2,2-dimethyl-7-methoxybenzofuran-4-carbonsäure

5,5 g 2,3-Dihydro-2,2-dimethyl-7-methoxybenzofuran-4-carbonsäuremethylester werden in einem Gemisch aus 150 ml Ethanol und 50 ml 2N NaOH 2 h bei 60°C gerührt. Das Ethanol wird abdestilliert, der Rückstand im Wasser aufgenommen und mit 2N HCl ein pH von 4 eingestellt. Das dabei ausfallende Produkt wird abgesaugt, mit Wasser gewaschen und getrocknet. Man erhält 4,7 g der Titelverbindung mit Fp. 147-149°C.

### A3: 2,3-Dihydro-2,2-dimethyl-7-methoxybenzofuran-4-carbonsäuremethylester

15,6 g 3-Hydroxy-4-methoxy-2-(2-methyl-2-propenyl)benzoesäuremethylester werden in 250 ml abs. Dichlormethan gelöst und die Lösung mit 3 ml konzentrierter Schwefelsäure versetzt. Das Gemisch wird 12 h bei RT gerührt, dann wird mit Wasser versetzt und in der wäßrigen Phase durch Zugabe von 2N NaOH ein pH von 5 eingestellt. Nach Abtrennen der organischen Phase wird die wäßrige Phase noch 2 x mit Ethylacetat extrahiert. Die vereinigten organischen Phasen werden eingeengt und der ölige Rückstand über eine Kieselgelsäule mit Ethylacetat/Petrolether (4:6) chromatographiert. Die chromatographisch reinen Fraktionen des Produktes mit Rf ~ 0,6 werden vereinigt und eingeengt. Man erhält 6,6 g der Titelverbindung mit Fp. 65-67°C.

### A4: 3-Hydroxy-4-methoxy-2-(2-methyl-2-propenyl)-benzoesäuremethylester

20 g 4-Methoxy-3-(2-methyl-2-propenyloxy)benzoesäuremethylester werden in 60 ml Chinolin gelöst und das Gemisch 2 h auf 180-190°C erhitzt. Nach Abkühlen wird mit Ethylacetat versetzt und das Chinolin mit 2N HCl extrahiert. Die organische Phase wird noch 2 x mit Wasser gewaschen und eingeengt. Der ölige Rückstand wird über eine Kieselgelsäule mit Ethylacetat/Petrolether (4:6) chromatographiert. Die chromatographisch reinen Fraktionen werden vereinigt, eingeengt und im Hochvakuum getrocknet. Man erhält 15,6 g der Titelverbindung als hellgelbes Öl.

### A5: 4-Methoxy-3-(2-methyl-2-propenyloxy)benzoesäuremethylester

22 g 3-Hydroxy-4-methoxybenzoesäuremethylester werden in 200 ml wasserfreiem DMF gelöst und dann 41 g gemahlenes Kaliumcarbonat und 14,7 ml 3-Chlor-2-methylpropen zugegeben. Das Gemisch wird 5 h bei 60°C gerührt. Nach Abkühlen wird abgesaugt, das Filtrat mit Wasser versetzt und dann 3 x mit Ethylacetat extrahiert. Der nach Einengen der Extrakte zurückbleibende Rückstand wird mit Petrolether kristallisiert. Man erhält 21 g der Titelverbindung mit Fp. 62-63°C.

### B1: 2,3-Dihydro-7-methoxybenzofuran-2-spiro-1'-cyclopentan-4-carbonsäurechlorid

Die Titelverbindung wird analog Ausgangsverbindung A1 aus 2,3-Dihydro-7-methoxy-benzofuran-2-spiro-1'-cyclopentan-4-carbonsäure in 50 ml abs. Toluol und 3 ml Thionylchlorid hergestellt und ohne weitere Reinigung weiter umgesetzt.

### B2: 2,3-Dihydro-7-methoxybenzofuran-2-spiro-1'-cyclopentan-4-carbonsäure

2,6 g 2,3-Dihydro-7-methoxybenzofuran-2-spiro-1'-cyclopentan-4-carbonsäuremethylester werden analog Beispiel A2 in 50 ml Ethanol und 10 ml 2N NaOH verseift. Man erhält 2,3 g der Titelverbindung mit Fp. 166-168°C,

### B3: 2,3-Dihydro-7-methoxybenzofuran-2-spiro-1'-cyclopentan-4-carbonsäuremethylester

10,2 g 2-Cyclopenten-1-ylmethyl-3-hydroxy-4-methoxy-benzoesäuremethylester werden in 500 ml wasserfreiem n-Hexan gelöst und mit ca. 5 g Amberlyst 15 versetzt. Das Gemisch wird 3 Tage bei RT gerührt, filtriert und eingeengt. Der Rückstand wird über eine Kieselgelsäule mit Ethylacetat/Petrolether (4:6) chromatographiert, die chromatographisch reinen Fraktionen vereinigt, eingeengt und im Hochvakuum getrocknet. Man erhält 7,2 g der Titelverbindung als gelbes Öl.

### B4: 2-Cyclopenten-1-ylmethyl-3-hydroxy-4-methoxybenzoesäuremethylester

12,7 g 3-(2-Methylencyclopentyloxy)-4-methoxybenzoesäuremethylester werden mit 50 ml Chinolin versetzt und das Gemisch 1 h bei 190°C gerührt. Nach Abkühlen wird mit Wasser versetzt, mit 2N HCl pH3 eingestellt und mit Ethylacetat extrahiert. Der nach Einengen des Lösungsmittel verbleibende Rückstand wird über eine Kieselgelsäule mit Ethylacetat/Petrolether (4:6) chromatographiert. Die chromatographisch reinen Fraktionen werden eingeengt und im Hochvakuum getrocknet. Man erhält 10,2 g der Titelverbindung als gelbes Öl.

### B5: 3-(2-Methylencyclopentyloxy)-4-methoxy-benzoesäuremethylester

28,5 g Methyl-triphenylphosphoniumbromid werden in 300 ml wasserfreiem THF unter Stickstoff suspendiert und das Gemisch auf -40°C abgekühlt. Dann werden unter Rühren 50 ml n-Butyllithium (1,6M) in n-Hexan zugetropft. Nach 30 min Rühren bei -20 bis -10°C wird eine Lösung von 20 g 4-Methoxy-3-(2-oxocyclopentyloxy)benzoesäuremethylester in 100 ml abs. THF zugetropft. Danach läßt man das Gemisch sich auf RT erwärmen und rührt noch 1 h. Es wird auf Wasser gegossen und mit Ethylacetat extrahiert. Das nach Einengen der organischen Phase verbleibende Öl wird über eine Kieselgelsäule mit Ethylacetat/Petrolether (4:6) chromatographiert. Die chromatographisch reinen Fraktionen werden vereinigt, eingeengt und im Hochvakuum getrocknet. Man erhält 12,7 g der Titelverbindung als farbloses Öl.

### B6: 4-Methoxy-3-(2-oxocyclopentyloxy)benzoesäuremethylester

23,8 g 3-Hydroxy-4-methoxy-benzoesäuremethylester werden in 200 ml wasserfreiem DMF gelöst und die Lösung mit 35 g Kaliumcarbonat (gemahlen) und 13 ml 2-Chlorcyclopentanon versetzt. Das Gemisch wird 3 h bei 60°C gerührt, dann vom Feststoff abgesaugt und das Filtrat im Vakuum eingeengt. Der Rückstand wird über eine Kieselgelsäule mit Ethylacetat/Petrolether (4:6) chromatographiert. Die chromatographisch reinen Fraktionen werden vereinigt, eingeengt und im Hochvakuum getrocknet. Man erhält 24,3 g der Titelverbindung als hellgelbes Öl.

### C1: 7-Difluormethoxy-2,3-dihydrobenzofuran-2-spiro-1'-cyclopentan-4-carbonsäurechlorid

Analog Beispiel A1 werden 0,7 g 7-Difluormethoxy-2,3-dihydrobenzofuran-2-spiro-1'-cyclopentan-4-carbonsäure in einem Gemisch aus 20 ml abs. Toluol und 2 ml Thionylchlorid umgesetzt und ohne weitere Reinigung weiterverarbeitet.

### C2: 7-Difluormethoxy-2,3-dihydrobenzofuran-2-spiro-1'-cyclopentan-4-carbonsäure

Analog Beispiel A2 erhält man aus 2,4 g 7-Difluormethoxy-2,3-dihydrobenzofuran-2-spiro-1'-cyclopentan-4-carbonsäureethylester 2 g der Titelverbindung mit Fp.: 143-145°C.

### C3: 7-Difluormethoxy-2,3-dihydrobenzofuran-2-spiro-1'-cyclopentan-4-carbonsäureethylester

2,7 g 2,3-Dihydro-7-hydroxybenzofuran-2-spiro-1'-cyclopentan-4-carbonsäureethylester werden in 70 ml Dioxan gelöst, 3 ml 50 % NaOH-Lösung und 0,1 g Benzyltrimethylammoniumchlorid zugegeben und dann in das Gemisch unter Rühren bei 70-75°C Difluorchlormethan bis zur Beendigung der Reaktion (ca. 1 h) eingeleitet. Nach Abkühlen wird auf Wasser gegossen und 3 x mit 100 ml Ethylacetat extrahiert. Nach Trocknen über Natriumsulfat wird eingeengt und der Rückstand über eine Kieselgelsäule mit Ethylacetat/Petrolether 4:6 chromatografiert. Die chromatografisch reinen Fraktionen werden vereinigt, eingeengt und im Hochvakuum getrocknet. Man erhält 2,4 g der Titelverbindung als hellgelbes Öl.

### C4: 2,3-Dihydro-7-hydroxybenzofuran-2-spiro-1'-cyclopentan-4-carbonsäureethylester

4,1 g 7-Benzyloxy-2,3-dihydrobenzofuran-2-spiro-1'-cyclopentan-4-carbonsäureethylester, 0,5 g Pd/C (10 %) und 20 ml Cyclohexan werden in 100 ml Toluol 4 h unter Rückfluß zum Sieden erhitzt. Nach Abkühlen wird filtriert und das Filtrat zur Trockene eingeengt. Man erhält 2,7 g der Titelverbindung als hellbraunes Öl.

### C5: 7-Benzyloxy-2,3-dihydrobenzofuran-2-spiro-1'-cyclopentan-4-carbonsäureethylester

10 g Methyltriphenylphosphoniumbromid-Natriumamid-Gemisch (FLUKA 69500) werden unter einer N₂-Atmosphäre in 100 ml abs. THF suspendiert und 0,5 h bei RT gerührt. Dann wird innerhalb von 30 min eine Lösung von 7 g 4-Benzyloxy-3-(2-oxocyclopentyloxy)benzoesäureethylester in 20 ml abs. THF zugetropft. Es wird 2 h bei RT gerührt, dann wird auf Wasser gegossen und 3 x mit 100 ml Ethylacetat extrahiert. Nach Trocknen über Natriumsulfat wird zur Trockene eingeengt. Zur Umlagerung wird der ölige Rückstand 1,5 h bei 190°C gerührt. Nach Abkühlen wird mit 100 ml Toluol versetzt, 10 g Amberlyst 15 (wasserfrei) zugegeben und das Gemisch 3 h bei 80°C gerührt. Danach wird filtriert, mit Methanol nachgewaschen und das Filtrat zur Trockene eingeengt. Der Rückstand wird über eine Kieselgelsäule mit Ethylacetat/Petrolether 4:6 chromatografiert. Die Fraktionen mit dem Hauptprodukt (R_{f} ~ 0,8) werden vereinigt, eingeengt und im Hochvakuum getrocknet. Man erhält 4,1 g der Titelverbindung als hellgelbes Öl.

### C6: 4-Benzyloxy-3-(2-oxocyclopentyloxy)benzoesäureethylester

Analog Beispiel B6 werden aus 34 g 4-Benzyloxy-3-hydroxybenzoesäureethylester, 35 g Kaliumcarbonat und 15 ml 2-Chlorcyclopentanon 36 g der Titelverbindung als farbloses Öl erhalten.

### D1: 2,3-Dihydro-7-methoxybenzofuran-2-spiro-1'-cyclohexan-4-carbonsäurechlorid

Analog Beispiel A1 werden 2 g 2,3-Dihydro-7-methoxybenzofuran-2-spiro-1'-c-yclohexan-4-carbonsäure in 50 ml Toluol mit 5 ml Thionylchlorid umgesetzt.

### D2: 2,3-Dihydro-7-methoxybenzofuran-2-spiro-1'-cyclohexan-4-carbonsäure

Analog Beispiel A1 erhält man aus 10,3 g 2,3-Dihydro-7-methoxybenzofuran-2-spiro-1'-cyclohexan-4-carbonsäuremethylester 9 g der Titelverbindung mit Fp.: 171-173°C.

### D3: 2,3-Dihydro-7-methoxybenzofuran-2-spiro-1'-cyclohexan-4-carbonsäuremethylester

Analog Beispiel B3 erhält man aus 17 g 2-Cyclohexen-1-ylmethyl-3-hydroxy-4-methoxy-benzoesäuremethylester in 500 ml n-Hexan und 15 g Amberlyst 15 (4 h bei 60°C) 10,3 g der Titelverbindung als gelbes Öl.

### D4: 2-Cyclohexen-1-ylmethyl-3-hydroxy-4-methoxy-benzoesäuremethylester

Analog Beispiel B4 erhält man aus 21 g 3-(2-Methylencyclohexyloxy)-4-methoxy-benzoesäuremethylester (Umsetzung 2 h bei 190°C) 17 g der Titelverbindung als gelbes Öl.

### D5: 3-(2-Methylencyclohexyloxy)-4-methoxy-benzoesäuremethylester

43,8 g Methyltriphenylphosphoniumbromid in 300 ml abs. Dimethoxyethan werden unter Stickstoff portionsweise mit 3,6 g Natriumhydrid (80 % in Paraffin) versetzt. Das Gemisch wird 1 h bei RT gerührt und dann eine Lösung von 30 g 4-Methoxy-3-(2-oxo-cyclohexyloxy)benzoesäuremethylester langsam zugetropft. Die Mischung wird über Nacht bei RT gerührt und dann analog Beispiel B5 aufgearbeitet. Man erhält 21 g der Titelverbindung als farbloses Öl.

### D6: 4-Methoxy-3-(2-oxocyclohexyloxy)benzoesäuremethylester

Analog Beispiel B6 erhält man aus 25 g 3-Hydroxy-4-methoxybenzoesäuremethylester, 41 g Kaliumcarbonat und 17,5 ml 2-Chlor-cyclohexanon in 200 ml DMF 32,9 g der Titelverbindung als hellgelbes Öl.

### E1: 2,3-Dihydro-7-methoxybenzofuran-2-spiro-1'-(4'-oxacyclohexan)-4-carbonsäurechlorid

Analog Beispiel A1 werden 1 g 2,3-Dihydro-7-methoxybenzofuran-2-spiro-1'-(4'-oxacyclohexan)-4-carbonsäure in einem Gemisch aus 50 ml Toluol und 5 ml Thionylchlorid umgesetzt und ohne weitere Reinigung weiterverarbeitet.

### E2: 2,3-Dihydro-7-methoxybenzofuran-2-spiro-1'-(4'-oxacyclohexan)-4-carbonsäure

Analog Beispiel A2 werden 1,3 g 2,3-Dihydro-7-methoxybenzofuran-2-spiro-1'-(4'-oxacyclohexan)-4-carbonsäuremethylester in einem Gemisch aus 50 ml Methanol und 10 ml 1 N Natronlauge verseift. Man erhält 1 g der Titelverbindung mit Fp.: 194-196°C.

### E3: 2,3-Dihydro-7-methoxybenzofuran-2-spiro-1'-(4'-oxacyclohexan)-4-carbonsäuremethylester

3,6 g 4-Methoxy-3-(4-methylen-tetrahydropyran-3-yloxy)benzoesäuremethylester werden in 50 ml Chinolin gelöst und 1 h bei 190-200°C gerührt. Nach Abkühlen wird auf Wasser gegossen, mit 2 N Salzsäure pH 3 eingestellt und 3 x mit Ethylacetat extrahiert. Nach Trocknen über Natriumsulfat wird im Vakuum zur Trockene eingeengt und der Rückstand (2,9 g) in 150 ml n-Hexan gelöst. Die Lösung wird mit 2,9 g Amberlyst 15 versetzt und 4 h bei 60°C kräftig gerührt. Dann wird filtriert, das Filtrat im Vakuum eingeengt und der ölige Rückstand über eine Kieselgelsäule mit Ethylacetat/Petrolether 4:6 chromatografiert. Die chromatografisch reinen Fraktionen werden vereinigt, eingeengt und im Hochvakuum getrocknet. Man erhält 1,3 g der Titelverbindung als hellgelbes Öl.

### E4: 4-Methoxy-3-(4-methylen-tetrahydropyran-3-yloxy)benzoesäuremethylester

18,2 g Methyl-triphenylphosphoniumbromid werden unter einer Stickstoffatmosphäre in 200 ml Dimethoxyethan suspendiert und dann portionsweise 1,5 g Natriumhydrid (80 % in Paraffin) zugegeben. Es wird 3 h bei RT gerührt und dann innerhalb von 30 min eine Lösung von 13 g 4-Methoxy-3-(4-oxotetrahydropyran-3-yloxy)benzoesäuremethylester zugetropft. Es wird über Nacht gerührt, dann auf Wasser gegossen und 3 x mit Ethylacetat extrahiert. Nach Trocknen über Natriumsulfat wird im Vakuum eingeengt und der Rückstand über eine Kieselgelsäule mit Ethylacetat/Petrolether 4:6 chromatografiert. Die chromatografisch reinen Fraktionen werden vereint, eingeengt und im Hochvakuum getrocknet. Man erhält 3,6 g der Titelverbindung als hellgelbes Öl.

### E5: 4-Methoxy-3-(4-oxotetrahydropyran-3-yloxy)-benzoesäuremethylester

Analog Beispiel B6 erhält man aus 36,4 g 3-Hydroxy-4-methoxybenzoesäuremethylester, 50 g Kaliumcarbonat und 27 g 3-Chlortetrahydropyran-4-on in 200 ml DMF 11,5 g der Titelverbindung als hellgelbes Öl.

### F1: 2,2-Diethyl-2,3-dihydro-7-methoxybenzofuran-4-carbonsäurechlorid

Analog Beispiel A1 werden 1,2 g 2,2-Diethyl-2,3-dihydro-7-methoxybenzofuran-4-carbonsäure in einem Gemisch aus 10 ml Toluol und 2 ml Thionylchlorid umgesetzt.

### F2: 2,2-Diethyl-2,3-dihydro-7-methoxybenzofuran-4-carbonsäure

1,5 g 2,2-Diethyl-2,3-dihydro-7-methoxybenzofuran-4-carbonsäuremethylester werden in einem Gemisch aus 20 ml Ethanol und 5 ml 2N Natronlauge analog Beispiel A2 verseift und aufgearbeitet. Man erhält 1,2 g der Titelverbindung mit Fp.: 152-154°C.

### F3: 2,2-Diethyl-2,3-dihydro-7-methoxybenzofuran-4-carbonsäuremethylester

10 g Methyltriphenylphosphoniumbromid-Natriumamidgemisch (FLUKA 69500) werden bei ca. 10°C unter Schutzgas (Stickstoff) in 100 ml abs. THF gegeben, auf RT erwärmt und ca. 30 min gerührt. Dann wird eine Lösung von 5,3 g 4-Methoxy-3-(1-methyl-2-oxobutoxy)benzoesäure-methylester zugetropft. Es wird 1 h bei RT gerührt, dann wird auf Wasser gegossen und 3 x mit ca. 50 ml Ethylacetat extrahiert. Die vereinigten Extrakte werden über Natriumsulfat getrocknet, eingeengt und der ölige Rückstand im Hochvakuum getrocknet. Das erhaltene Öl (3,8 g) wird 1 h bei 190-200°C gerührt, abgekühlt und in 100 ml Toluol gelöst. Die Lösung wird mit 5 g Amberlyst 15 versetzt und über Nacht bei 80°C kräftig gerührt. Dann wird abfiltriert, eingeengt und der Rückstand über eine Kieselgelsäule mit Ethylacetat/Petrolether 4:6 chromatografiert. Die chromatografisch reinen Fraktionen werden vereinigt, eingeengt und der Rückstand im Hochvakuum getrocknet. Man erhält 1,5 g der Titelverbindung als hellgelbes Öl.

### F4: 4-Methoxy-3-(1-methyl-2-oxobutoxy)benzoesäuremethylester

Analog Beispiel B6 erhält man aus 48,5 g 3-Hydroxy-4-methoxybenzoesäuremethylester, 83 g Kaliumcarbonat und 43,9 g 2-Brom-pentan-3-on in 200 ml DMF 68 g der Titelverbindung mit Fp.: 63-65°C (Verrühren mit Petrolether).

### Gewerbliche Anwendbarkeit

Die erfindungsgemäßen Verbindungen besitzen wertvolle pharmakologische Eigenschaften, die sie gewerblich verwertbar machen. Als Zyklisch-Nukleotid Phosphodiesterase (PDE) Inhibitoren (und zwar des Typs IV) eignen sie sich einerseits als Bronchialtherapeutika (zur Behandlung von Atemwegsobstruktionen aufgrund ihrer dilatierenden aber auch aufgrund ihrer atemfrequenz-bzw. atemantriebssteigernden Wirkung), andererseits jedoch vor allem zur Behandlung von Erkrankungen, insbesondere entzündlicher Natur, z.B. der Atemwege (Asthma-Prophylaxe), der Haut, des Darm, der Augen und der Gelenke, die vermittelt werden durch Mediatoren, wie Histamin, PAF (Plättchenaktivierender Faktor), Arachidonsäure-Abkömmlinge wie Leukotriene und Prostaglandine, Zytokine, Interleukine IL-1 bis IL-12, alpha-, beta- und gamma-Interferon, Tumornekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz; oder Erkrankungen, die aufgrund der gewebsrelaxierenden Wirkung der PDE-Hemmstoffe behandelt werden können, wie beispielsweise Koliken der Nieren und der Harnleiter im Zusammenhang mit Nierensteinen; oder auch Erkrankungen des ZNS, wie beispielsweise Depressionen oder arteriosklerotische Demenz.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 0,5 mg/kg. Die übliche Dosis bei systemischer Therapie liegt zwischen 0,05 und 2 mg/kg pro Tag.

### Biologische Untersuchungen

Bei der Untersuchung der PDE IV-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminol-verstärkte Chemolumineszenz gemessen werden kann. (Mc Phail LC, Strum SL, Leone PA und Sozzani S, The neutrophil respiratory burst mechanism. In "Immunology Series" 57: 47-76, 1992; ed. Coffey RG (Marcel Decker, Inc., New York-Basel -Hong Kong)).

Substanzen, welche die Chemolumineszenz sowie die Zytokinsekretion und die Sekretion entzündungssteigernder Mediatoren an Entzündungszellen, insbesonders neutrophilen und eosinophilen Granulozyten hemmen, sind solche, welche die PDE IV hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE IV-Hemmung durch die erfindungsgemäßen Substanzen ist damit ein zentraler Indikator für die Unterdrückung von entzündlichen Prozessen. (Giembycz MA, Could isoenzyme-selective phosphodiesterase inhibitors render bronchodilatory therapy redundant in the treatment of bronchial asthma?. Biochem Pharmacol 43: 2041-2051, 1992; Torphy TJ et al., Phosphodiesterase inhibitors: new opportunities for treatment of asthma. Thorax 46: 512-523, 1991; Schudt C et al., Zardaverine: a cyclic AMP PDE III/IV inhibitor. In "New Drugs for Asthma Therapy", 379-402, Birkhäuser Verlag Basel 1991; Schudt C et al., Influence of selective phosphodiesterase inhibitors on human neutrophil functions and levels of cAMP and Caᵢ. Naunyn-Schmiedebergs Arch Pharmacol 344: 682-690, 1991; Nielson CP et al., Effects of selective phosphodiesterase inhibitors on polymorphonuclear leukocyte respiratory burst. J Allergy Clin Immunol 86: 801-808, 1990; Schade et al., The specific type III and IV phosphodiesterase inhibitor zardaverine suppresses formation of tumor necrosis factor by macrophages. European Journal of Pharmacology 230: 9-14, 1993).

### 1. Hemmung der PDE IV-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. 311, 193-198, 1980). Hierbei erfolgt im ersten Schritt die PDE-Reaktion. In einem zweiten Schritt wird das entstandene 5'-Nukleotid durch eine 5'-Nukleotidase des Schlangengiftes von Ophiophagus hannah (King Cobra) zum ungeladenen Nukleosid gespalten. Im dritten Schritt wird das Nukleosid auf Ionenaustauschsäulen vom verbliebenen geladenen Substrat getrennt. Die Säulen werden mit 2 ml 30 mM Ammonium formiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte ergeben sich aus der folgenden Tabelle A, in der die Nummern der Verbindungen den Nummern der Beispiele entsprechen.

**Tabelle A**

| Hemmung der PDE IV-Aktivität | |
|---|---|
| Verbindung | -log IC₅₀ |
| 1 | 8,47 |
| 2 | 9,42 |
| 3 | 9,87 |
| 4 | 9,09 |
| 5 | 8,57 |
| 6 | 8,63 |
| 7 | 8,57 |
| 8 | 8,42 |

### FORMELBLATT I

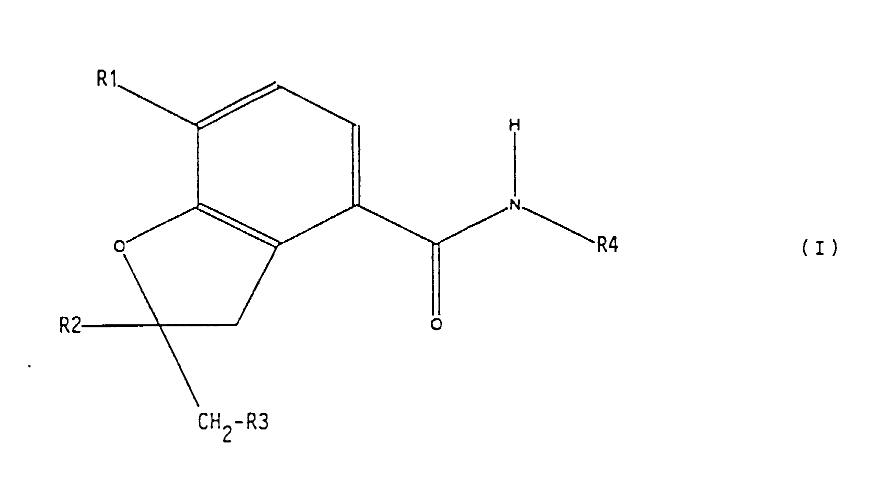

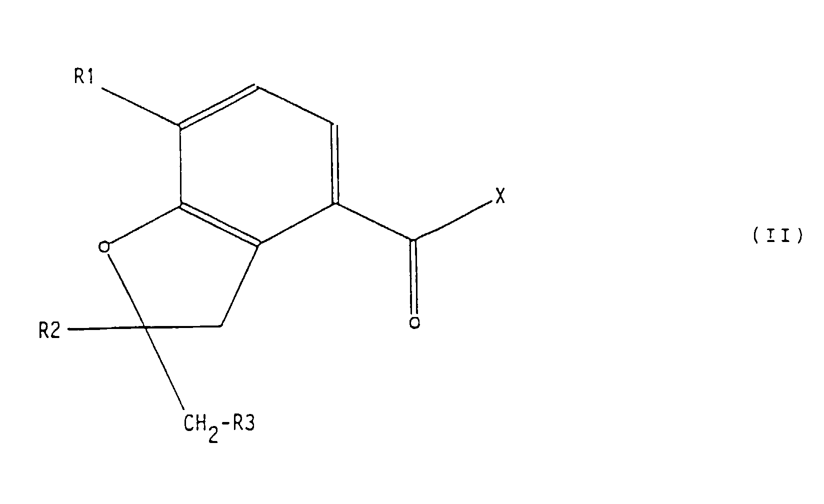

### FORMELBLATT II

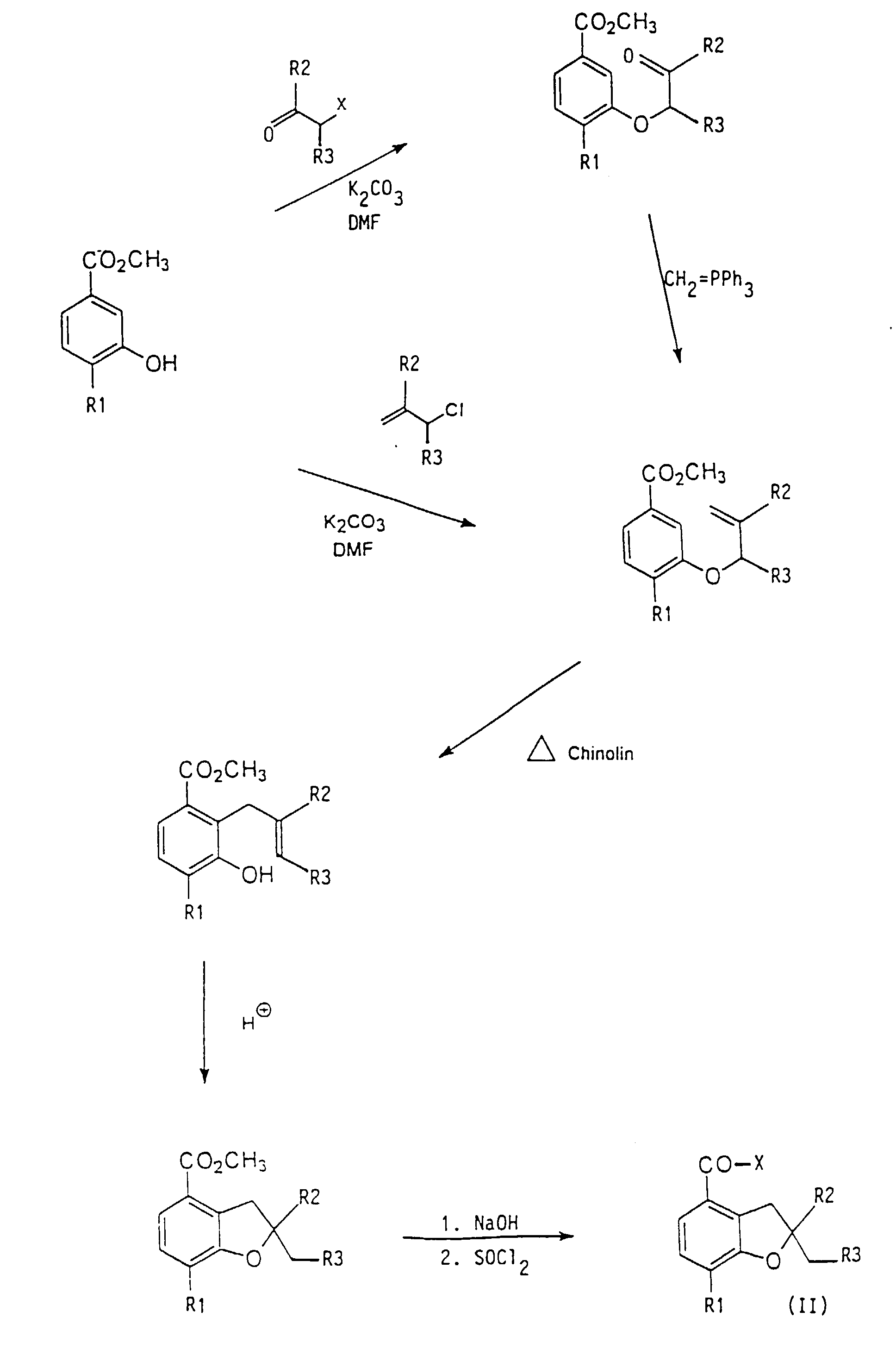

## Patentansprüche

1. Verbindungen der Formel I 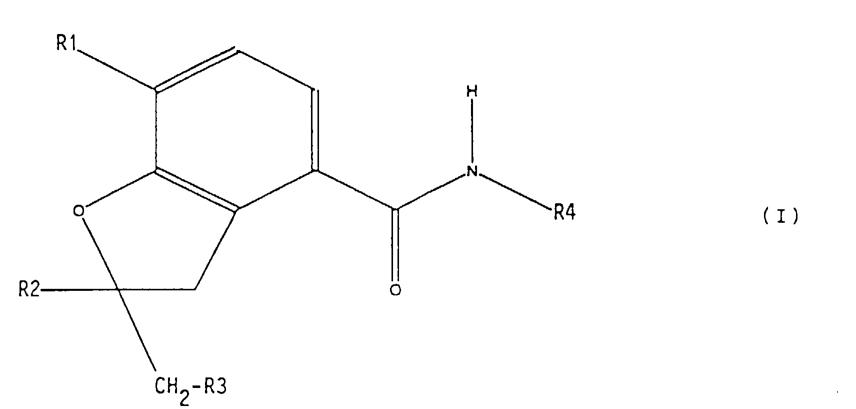 worin
R1 1-6C-Alkoxy, 3-7C-Cycloalkoxy, 3-7C-Cycloalkylmethoxy, Benzyloxy oder ganz oder teilweise durch Fluor substituiertes 1-4C-Alkoxy bedeutet,
R2 1-4C-Alkyl und
R3 Wasserstoff oder 1-4C-Alkyl bedeutet,
oder
R2 und R3 gemeinsam und unter Einschluß der beiden Kohlenstoffatome, an die sie gebunden sind, einen 5-, 6- oder 7-gliedrigen, gewünschtenfalls durch ein Sauerstoffatom unterbrochenen Kohlenwasserstoffring darstellen,
R4 Phenyl, Pyridyl, durch R41, R42 und R43 substituiertes Phenyl oder durch R44, R45, R46 und R47 substituiertes Pyridyl bedeutet, wobei
R41 Hydroxy, Halogen, Cyano, Carboxyl, Trifluormethyl, 1-4C-Alkyl, 1-4C-Alkoxy, 1-4C-Alkoxycarbonyl, 1-4C-Alkylcarbonyl, 1-4C-Alkylcarbonyloxy, Amino, Mono- oder Di-1-4C-alkylamino oder 1-4C-Alkylcarbonylamino,
R42 Wasserstoff, Hydroxy, Halogen, Amino, Trifluormethyl, 1-4C-Alkyl oder 1-4C-Alkoxy,
R43 Wasserstoff, Halogen, 1-4C-Alkyl oder 1-4C-Alkoxy,
R44 Hydroxy, Halogen, Cyano, Carboxyl, 1-4C-Alkyl, 1-4C-Alkoxy, 1-4C-Alkoxycarbonyl oder Amino,
R45 Wasserstoff, Halogen, Amino oder 1-4C-Alkyl,
R46 Wasserstoff oder Halogen und
R47 Wasserstoff oder Halogen bedeutet,
die Salze dieser Verbindungen sowie die N-Oxide der Pyridine und deren Salze.

2. Verbindungen der Formel I nach Anspruch 1, in denen
R1 1-4C-Alkoxy, 3-5C-Cycloalkoxy, 3-5C-Cycloalkylmethoxy oder ganz oder teilweise durch Fluor substituiertes 1-4C-Alkoxy bedeutet,
R2 1-4C-Alkyl und
R3 Wasserstoff oder 1-4C-Alkyl bedeutet,
oder
R2 und R3 gemeinsam und unter Einschluß der beiden Kohlenstoffatome, an die sie gebunden sind, einen Cyclopentan-, Cyclohexan-, Tetrahydrofuranoder Tetrahydropyranring darstellen,
R4 Phenyl, Pyridyl, durch R41, R42 und R43 substituiertes Phenyl oder durch R44, R45, R46 und R47 substituiertes Pyridyl bedeutet, wobei
R41 Halogen, Cyano, Carboxyl, 1-4C-Alkyl, 1-4C-Alkoxy oder 1-4C-Alkoxycarbonyl,
R42 Wasserstoff, Halogen, 1-4C-Alkyl oder 1-4C-Alkoxy,
R43 Wasserstoff, Halogen, 1-4C-Alkyl oder 1-4C-Alkoxy,
R44 Halogen oder 1-4C-Alkyl,
R45 Wasserstoff oder Halogen,
R46 Wasserstoff oder Halogen und
R47 Wasserstoff oder Halogen bedeutet,
die Salze dieser Verbindungen sowie die N-Oxide der Pyridine und deren Salze.

3. Verbindungen der Formel I nach Anspruch 1, in denen
R1 1-4C-Alkoxy, 3-5C-Cycloalkoxy, 3-5C-Cycloalkylmethoxy oder ganz oder teilweise durch Fluor substituiertes 1-4C-Alkoxy bedeutet,
R2 1-4C-Alkyl und
R3 Wasserstoff oder 1-4C-Alkyl bedeutet,
oder
R2 und R3 gemeinsam und unter Einschluß der beiden Kohlenstoffatome, an die sie gebunden sind, einen Cyclopentan-, Cyclohexan-, Tetrahydrofuranoder Tetrahydropyranring darstellen,
R4 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2-Chlor-6-fluorphenyl, 2,6-Difluorphenyl, 2,6-Dichlorphenyl, 3,5-Dichlorpyrid-4-yl, 3-Methylpyrid-2-yl, 2-Chlorpyrid-3-yl, 3,5-Dibrompyrid-2-yl, 3,5-Difluorpyrid-4-yl, 2-Chlorphenyl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

4. Verbindungen der Formel I nach Anspruch 1, in denen
R1 Methoxy, Ethoxy, Cyclopropylmethoxy, Difluormethoxy, Trifluormethoxy oder 2,2,2-Trifluorethoxy bedeutet,
R2 Methyl oder Ethyl und
R3 Wasserstoff oder Methyl bedeutet,
oder
R2 und R3 gemeinsam und unter Einschluß der beiden Kohlenstoffatome, an die sie gebunden sind, einen Cyclopentan-, Cyclohexan-, Tetrahydrofuran-oder Tetrahydropyranring darstellen,
R4 2-Bromphenyl, 2,6-Dichlor-4-ethoxycarbonylphenyl, 2,6-Dimethoxyphenyl, 4-Cyano-2-fluorphenyl, 2,4,6-Trifluorphenyl, 2-Chlor-6-methylphenyl, 2,6-Dimethylphenyl, 2-Chlor-6-fluorphenyl, 2,6-Difluorphenyl, 2,6-Dichlorphenyl, 3,5-Dichlorpyrid-4-yl, 3-Methylpyrid-2-yl, 2-Chlorpyrid-3-yl, 3,5-Dibrompyrid-2-yl, 3,5-Difluorpyrid-4-yl, 2-Chlorphenyl, 2,3,5,6-Tetrafluorpyrid-4-yl, 3-Chlor-2,5,6-trifluorpyrid-4-yl, 3,5-Dichlor-2,6-difluorpyrid-4-yl oder 2,6-Dichlorpyrid-3-yl bedeutet,
die Salze dieser Verbindungen sowie die N-Oxide der Pyridine und deren Salze.

5. Verbindungen der Formel I nach Anspruch 1, in denen
R1 Methoxy, Ethoxy, Cyclopropylmethoxy, Difluormethoxy, Trifluormethoxy oder 2,2,2-Trifluorethoxy bedeutet,
R2 Methyl oder Ethyl und
R3 Wasserstoff oder Methyl bedeutet,
oder
R2 und R3 gemeinsam und unter Einschluß der beiden Kohlenstoffatome, an die sie gebunden sind, einen Cyclopentan-, Cyclohexan-, Tetrahydrofuran- oder Tetrahydropyranring darstellen,
R4 3,5-Dichlorpyrid-4-yl, 2,6-Dichlorphenyl oder 2,6-Difluorphenyl bedeutet, die Salze dieser Verbindungen sowie die N-Oxide der Pyridine und deren Salze.

6. Verfahren zur Herstellung der Verbindungen der Formel I nach Anspruch 1 und ihrer Salze, sowie der N-Oxide der Pyridine und deren Salze, dadurch gekennzeichnet, daß man Verbindungen der Formel II, 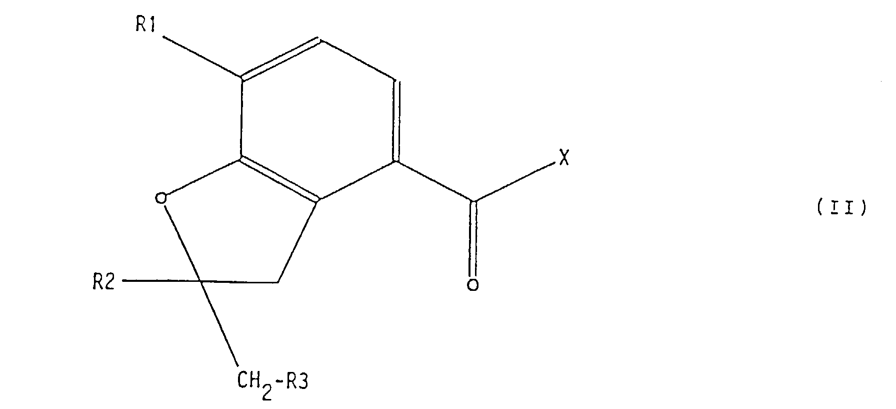 in denen R1, R2 und R3 die in Anspruch 1 angegebenen Bedeutungen haben und X eine geeignete Abgangsgruppe darstellt, mit Aminen R4-NH₂ umsetzt, und daß man gewünschtenfalls anschließend erhaltene Verbindungen der Formel I in ihre Salze und/oder erhaltene Pyridine in die N-Oxide und gewünschtenfalls anschließend in die Salze überführt, oder daß man gewünschtenfalls anschließend erhaltene Salze der Verbindungen der Formel I in die freien Verbindungen überführt.

7. Arzneimittel enthaltend eine oder mehrere Verbindungen nach Anspruch 1 zusammen mit üblichen pharmazeutischen Hilfs- und/oder Trägerstoffen.

8. Verbindungen nach Anspruch 1 zur Anwendung bei der Behandlung von Krankheiten.

9. Verwendung von Verbindungen nach Anspruch 1 zur Herstellung von Arzneimitteln für die Behandlung von Atemwegserkrankungen.

10. Verwendung von Verbindungen nach Anspruch 1 zur Herstellung von Arzneimitteln für die Behandlung von Dermatosen.

## Claims

1. Compounds of the formula I 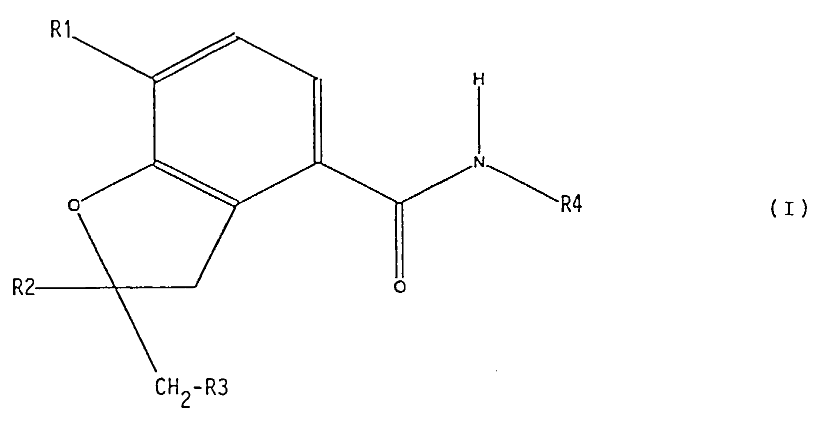 in which
R1 is 1-6C-alkoxy, 3-7C-cycloalkoxy, 3-7C-cycloalkylmethoxy, benzyloxy or completely or partially fluorine-substituted 1-4C-alkoxy,
R2 is 1-4C-alkyl and
R3 is hydrogen or 1-4C-alkyl,
or
R2 and R3, together and including the two carbon atoms to which they are bonded, are a 5-, 6-or 7-membered hydrocarbon ring, if desired interrupted by an oxygen atom,
R4 is phenyl, pyridyl, R41-, R42- and R43-substituted phenyl or R44-, R45-, R46- and R47-substituted pyridyl, where
R41 is hydroxyl, halogen, cyano, carboxyl, trifluoromethyl, 1-4C-alkyl, 1-4C-alkoxy, 1-4C-alkoxycarbonyl, 1-4C-alkylcarbonyl, 1-4C-alkylcarbonyloxy, amino, mono- or di-1-4C-alkylamino or 1-4C-alkylcarbonylamino,
R42 is hydrogen, hydroxyl, halogen, amino, trifluoromethyl, 1-4C-alkyl or 1-4C-alkoxy,
R43 is hydrogen, halogen, 1-4C-alkyl or 1-4C-alkoxy,
R44 is hydroxyl, halogen, cyano, carboxyl, 1-4C-alkyl, 1-4C-alkoxy, 1-4C-alkoxycarbonyl or amino,
R45 is hydrogen, halogen, amino or 1-4C-alkyl,
R46 is hydrogen or halogen and
R47 is hydrogen or halogen,
the salts of these compounds, and the N-oxides of the pyridines and their salts.

2. Compounds of the formula I as claimed in claim 1, in which
R1 is 1-4C-alkoxy, 3-5C-cycloalkoxy, 3-5C-cycloalkylmethoxy or completely or partially fluorine-substituted 1-4C-alkoxy,
R2 is 1-4C-alkyl and
R3 is hydrogen or 1-4C-alkyl,
or
R2 and R3, together and including the two carbon atoms to which they are bonded, are a cyclopentane, cyclohexane, tetrahydrofuran or tetrahydropyran ring,
R4 is phenyl, pyridyl, R41-, R42- and R43-substituted phenyl or R44-, R45-, R46- and R47-substituted pyridyl, where
R41 is halogen, cyano, carboxyl, 1-4C-alkyl, 1-4C-alkoxy or 1-4C-alkoxycarbonyl,
R42 is hydrogen, halogen, 1-4C-alkyl or 1-4C-alkoxy,
R43 is hydrogen, halogen, 1-4C-alkyl or 1-4C-alkoxy,
R44 is halogen or 1-4C-alkyl,
R45 is hydrogen or halogen,
R46 is hydrogen or halogen and
R47 is hydrogen or halogen,
the salts of these compounds, and the N-oxides of the pyridines and their salts.

3. Compounds of the formula I as claimed in claim 1 in which
R1 is 1-4C-alkoxy, 3-5C-cycloalkoxy, 3-5C-cycloalkylmethoxy or completely or partially fluorine-substituted 1-4C-alkoxy,
R2 is 1-4C-alkyl and
R3 is hydrogen or 1-4C-alkyl,
or
R2 and R3, together and including the two carbon atoms to which they are bonded, are a cyclopentane, cyclohexane, tetrahydrofuran or tetrahydropyran ring,
R4 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2-chloro-6-fluorophenyl, 2,6-difluorophenyl, 2,6-dichlorophenyl, 3,5-dichloropyrid-4-yl, 3-methylpyrid-2-yl, 2-chloropyrid-3-yl, 3,5-dibromopyrid-2-yl, 3,5-difluoropyrid-4-yl, 2-chlorophenyl, 2,3,5,6-tetrafluoropyrid-4-yl, 3-chloro-2,5,6-trifluoropyrid-4-yl, 3,5-dichloro-2,6-difluoropyrid-4-yl or 2,6-dichloropyrid-3-yl,
the salts of these compounds, and the N-oxides of the pyridines and their salts.

4. Compounds of the formula I as claimed in claim 1 in which
R1 is methoxy, ethoxy, cyclopropylmethoxy, difluoromethoxy, trifluoromethoxy or 2,2,2-trifluoroethoxy,
R2 is methyl or ethyl and
R3 is hydrogen or methyl,
or
R2 and R3, together and including the two carbon atoms to which they are bonded, are a cyclopentane, cyclohexane, tetrahydrofuran or tetrahydropyran ring,
R4 is 2-bromophenyl, 2,6-dichloro-4-ethoxycarbonylphenyl, 2,6-dimethoxyphenyl-, 4-cyano-2-fluorophenyl, 2,4,6-trifluorophenyl, 2-chloro-6-methylphenyl, 2,6-dimethylphenyl, 2-chloro-6-fluorophenyl, 2,6-difluorophenyl, 2,6-dichlorophenyl, 3,5-dichloropyrid-4-yl, 3-methylpyrid-2-yl, 2-chloropyrid-3-yl, 3,5-dibromopyrid-2-yl, 3,5-difluoropyrid-4-yl, 2-chlorophenyl, 2,3,5,6-tetrafluoropyrid-4-yl, 3-chloro-2,5,6-trifluoropyrid-4-yl, 3,5-dichloro-2,6-difluoropyrid-4-yl or 2,6-dichloropyrid-3-yl,
the salts of these compounds, and the N-oxides of the pyridines and their salts.

5. Compounds of the formula I as claimed in claim 1 in which
R1 is methoxy, ethoxy, cyclopropylmethoxy, difluoromethoxy, trifluoromethoxy or 2,2,2-trifluoroethoxy,
R2 is methyl or ethyl and
R3 is hydrogen or methyl,
or
R2 and R3, together and including the two carbon atoms to which they are bonded, are a cyclopentane, cyclohexane, tetrahydrofuran or tetrahydropyran ring,
R4 is 3,5-dichloropyrid-4-yl, 2,6-dichlorophenyl or 2,6-difluorophenyl,
the salts of these compounds, and the N-oxides of the pyridines and their salts.

6. A process for the preparation of the compounds of the formula I as claimed in claim 1 and their salts, and also the N-oxides of the pyridines and their salts, characterized in that compounds of the formula II 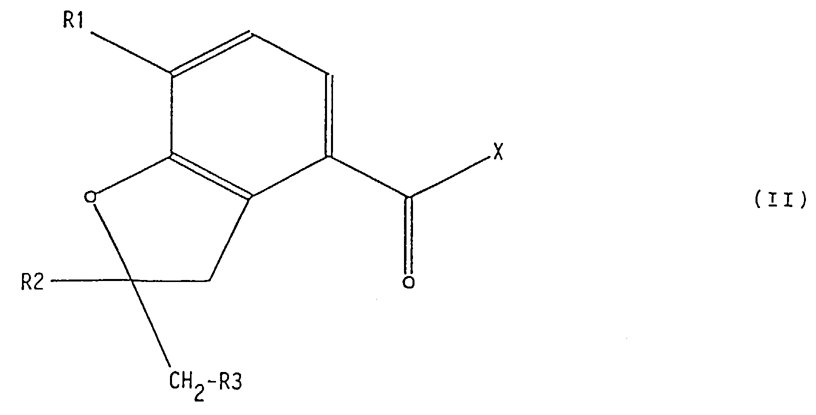 in which R1, R2 and R3 have the meanings indicated in claim 1 and X is a suitable leaving group are reacted with amines R4-NH₂, and in that if desired, compounds of the formula I obtained are then converted into their salts and/or pyridines obtained are then converted into the N-oxides and, if desired, then into the salts, or in that, if desired, salts of the compounds of the formula I obtained are then converted into the free compounds.

7. Medicaments containing one or more compounds as claimed in claim 1 together with customary pharmaceutical auxiliaries and/or excipients.

8. A compound as claimed in claim 1 for use in the treatment of diseases.

9. The use of compounds as claimed in claim 1 for the production of medicaments for the treatment of airway disorders.

10. The use of compounds as claimed in claim 1 for the production of medicaments for the treatment of dermatoses.

## Revendications

1. Composés de formule I 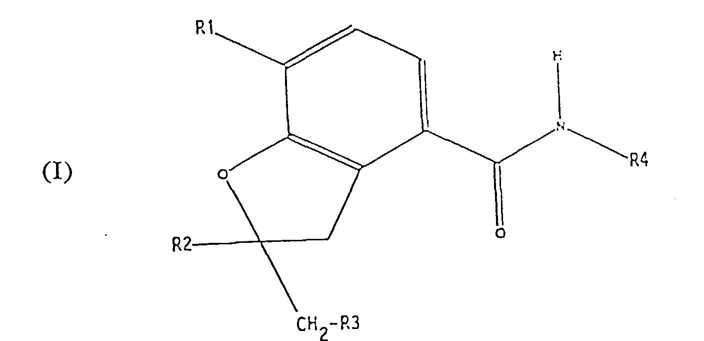 dans laquelle
R¹ représente un groupe alcoxy en C₁₋₆, cycloalcoxy en C₃₋₇, (cycloalkyle en C₃₋₇)-méthoxy, benzyloxy ou alcoxy en C₁₋₄ totalement ou partiellement fluoré,
R² représente un groupe alkyle en C₁₋₄,
R³ représente un atome d'hydrogène ou un groupe alkyle en C₁₋₄,
ou R² et R³ représentent conjointement avec les deux atomes de carbone auxquels ils sont liés un cycle hydrocarboné à 5, 6 ou 7 chaînons éventuellement interrompu par un atome d'oxygène,
R⁴ représente un groupe phényle, pyridyle, phényle portant des substituants R⁴¹, R⁴² et R⁴³ ou pyridyle portant des substituants R⁴⁴, R⁴⁵, R⁴⁶ et R⁴⁷, où
R⁴¹ représente un groupe hydroxy, halogéno, cyano, carboxyle, trifluorométhyle, alkyle en C₁₋₄, alcoxy en C₁₋₄, (alcoxy en C₁₋₄)-carbonyle, (alkyle en C₁₋₄)-carbonyle, (alkyle en C₁₋₄)-carbonyloxy, amino, mono- ou di(alkyle en C₁₋₄)-amino ou (alkyle en C₁₋₄)-carbonylamino,
R⁴² représente un atome d'hydrogène ou d'halogène, un groupe hydroxy, amino, trifluorométhyle, alkyle en C₁₋₄ ou alcoxy en C₁₋₄,
R⁴³ représente un atome d'hydrogène ou d'halogène, un groupe alkyle en C₁₋₄ ou alcoxy en C₁₋₄,
R⁴⁴ représente un groupe hydroxy, halogéno, cyano, carboxyle, alkyle en C₁₋₄, alcoxy en C₁₋₄, (alcoxy en C₁₋₄)-carbonyle ou amino,
R⁴⁵ représente un atome d'hydrogène ou d'halogène, un groupe amino ou alkyle en C₁₋₄,
R⁴⁶ représente un atome d'hydrogène ou d'halogène et
R⁴⁷ représente un atome d'hydrogène ou d'halogène,
les sels de ces composés ainsi que les N-oxydes des pyridines et les sels correspondants.

2. Composés de formule (I) selon la revendication 1, dans lesquels
R¹ représente un groupe alcoxy en C₁₋₄, cycloalcoxy en C₃₋₅, (cycloalkyle en C₃₋₅)-méthoxy ou alcoxy en C₁₋₄ totalement ou partiellement fluoré,
R² représente un groupe alkyle en C₁₋₄,
R³ représente un atome d'hydrogène ou un groupe alkyle en C₁₋₄,
ou R² et R³ représentent conjointement avec les deux atomes de carbone auxquels ils sont liés un cycle cyclopentane, cyclohexane, tétrahydrofurane ou tétrahydropyrane,
R⁴ représente un groupe phényle, pyridyle, phényle portant des substituants R⁴¹, R⁴² et R⁴³ ou pyridyle portant des substituants R⁴⁴, R⁴⁵, R⁴⁶ et R⁴⁷, où
R⁴¹ représente un atome d'halogène, un groupe cyano, carboxyle, alkyle en C₁₋₄, alcoxy en C₁₋₄ ou (alcoxy en C₁₋₄)-carbonyle,
R⁴² représente un atome d'hydrogène ou d'halogène, un groupe alkyle en C₁₋₄ ou alcoxy en C₁₋₄,
R⁴³ représente un atome d'hydrogène ou d'halogène, un groupe alkyle en C₁₋₄ ou alcoxy en C₁₋₄,
R⁴⁴ représente un atome d'hydrogène ou un groupe alkyle en C₁₋₄,
R⁴⁵ représente un atome d'hydrogène ou d'halogène,
R⁴⁶ représente un atome d'hydrogène ou d'halogène et
R⁴⁷ représente un atome d'hydrogène ou d'halogène,
les sels de ces composés ainsi que les N-oxydes des pyridines et les sels correspondants.

3. Composés de formule (I) selon la revendication 1, dans lesquels
R¹ représente un groupe alcoxy en C₁₋₄, cycloalcoxy en C₃₋₅, (cycloalkyle en C₃₋₅)-méthoxy ou alcoxy en C₁₋₄ totalement ou partiellement fluoré,
R² représente un groupe alkyle en C₁₋₄,
R³ représente un atome d'hydrogène ou un groupe alkyle en C₁₋₄, ou R² et R³ représentent conjointement avec les deux atomes de carbone auxquels ils sont liés un cycle cyclopentane, cyclohexane, tétrahydrofurane ou tétrahydropyrane,
R⁴ représente un groupe 2-bromophényle, 2,6-dichloro-4-éthoxycarbonylphényle, 2,6-diméthoxyphényle, 4-cyano-2-fluorophényle, 2,4,6-trifluorophényle, 2-chloro-6-méthylphényle, 2,6-diméthylphényle, 2-chloro-6-fluorophényle, 2,6-difluorophényle, 2,6-dichlorophényle, 3,5-dichloropyrid-4-yle, 3-méthylpyrid-2-yle, 2-chloropyrid-3-yle, 3,5-dibromopyrid-2-yle, 3,5-difluoropyrid-4-yle, 2-chlorophényle, 2,3,5,6-tétrafluoropyrid-4-yle, 3-chloro-2,5,6,-trifluoropyrid-4-yle, 3,5-dichloro-2,6-difluoropyrid-4-yle ou 2,6-dichloropyrid-3-yle,
les sels de ces composés ainsi que les N-oxydes des pyridines et les sels correspondants.

4. Composés de formule (I) selon la revendication 1, dans lesquels
R1 représente un groupe méthoxy, éthoxy, cyclopropylméthoxy, difluorométhoxy, trifluorométhoxy ou 2,2,2-trifluoroéthoxy,
R2 représente un groupe méthyle ou éthyle,
R3 représente un atome d'hydrogène ou un groupe méthyle
ou R² et R³ représentent conjointement avec les deux atomes de carbone auxquels ils sont liés un cycle cyclopentane, cyclohexane, tétrahydrofurane ou tétrahydropyrane,
R⁴ représente un groupe 2-bromophényle, 2,6-dichloro-4-éthoxycarbonylphényle, 2,6-diméthoxyphényle, 4-cyano-2-fluorophényle, 2,4,6-trifluorophényle, 2-chloro-6-méthylphényle, 2,6-diméthylphényle, 2-chloro-6-fluorophényle, 2,6-difluorophényle, 2,6-dichlorophényle, 3,5-dichloropyrid-4-yle, 3-méthylpyrid-2-yle, 2-chloropyrid-3-yle, 3,5-dibromopyrid-2-yle, 3,5-difluoropyrid-4-yle, 2-chlorophényle, 2,3,5,6-tétrafluoropyrid-4-yle, 3-chloro-2,5,6,-trifluoropyrid-4-yle, 3,5-dichloro-2,6-difluoropyrid-4-yle ou 2,6-dichloropyrid-3-yle,
les sels de ces composés ainsi que les N-oxydes des pyridines et les sels correspondants.

5. Composés de formule (I) selon la revendication 1, dans lesquels
R1 représente un groupe méthoxy, éthoxy, cyclopropylméthoxy, difluorométhoxy, trifluorométhoxy ou 2,2,2-trifluoroéthoxy,
R2 représente un groupe méthyle ou éthyle,
R3 représente un atome d'hydrogène ou un groupe méthyle
ou R² et R³ représentent conjointement avec les deux atomes de carbone auxquels ils sont liés un cycle cyclopentane, cyclohexane, tétrahydrofurane ou tétrahydropyrane,
R4 représente un groupe 3,5-dichloropyrid-4-yle, 2,6-dichlorophényle ou 2,6-difluorophényle,
les sels de ces composés ainsi que les N-oxydes des pyridines et les sels correspondants.

6. Procédé de préparation des composés de formule (I) selon la revendication 1 et de leurs sels, ainsi que des N-oxydes des pyridines et de leurs sels, caractérisé par le fait que l'on fait réagir des composés de formule (II) 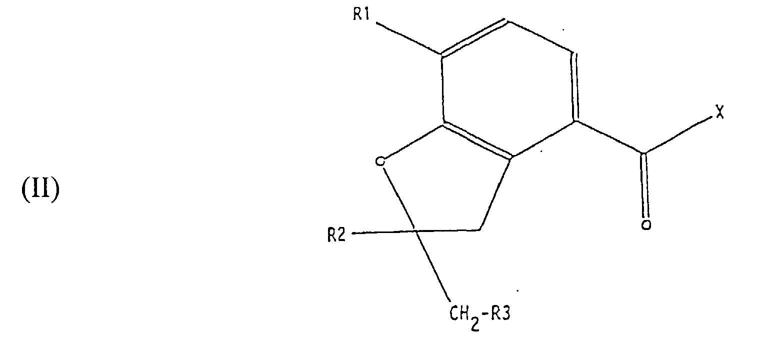 dans lesquels R¹, R² et R³ ont la signification indiquée dans la revendication 1 et X représente un groupe partant approprié, avec des amines de formule R⁴-NH₂, et que l'on convertit ensuite éventuellement les composés de formule (I) obtenus en leurs sels et/ou les pyridines obtenues en leur oxydes, puis ceux-ci éventuellement en leurs sels, ou que l'on convertit éventuellement ensuite des sels des composés de formule (I) obtenus en les composés libres correspondants.

7. Médicament contenant un ou plusieurs composés selon la revendication 1 en combinaison avec des excipients et/ou adjuvants pharmaceutiques usuels.

8. Composés selon la revendication 1 pour l'utilisation dans le traitement de maladies.

9. Utilisation de composés selon la revendication 1 pour la préparation de médicaments destinés au traitement de maladies des voies respiratoires.

10. Utilisation de composés selon la revendication 1 pour la préparation de médicaments destinés au traitement de dermatoses.
